(19) Europäisches Patentamt
European Patent Office
Office européen des brevets

(11) EP 1 318 991 B1

(12) **EUROPEAN PATENT SPECIFICATION**

(45) Date of publication and mention of the grant of the patent:
**16.08.2006 Bulletin 2006/33**

(21) Application number: **01973891.3**

(22) Date of filing: **21.09.2001**

(51) Int Cl.:
*C07D 307/32* (2006.01) *A61K 31/365* (2006.01)
*A61P 35/00* (2006.01)

(86) International application number:
**PCT/CA2001/001331**

(87) International publication number:
**WO 2002/024674 (28.03.2002 Gazette 2002/12)**

(54) **BENZOIC ACID DERIVATIVES AND USES THEREOF**

BENZOESAÜRE DERIVATE UND DEREN VERWENDUNG

DERIVES D'ACIDE BENZOIQUE ET LEURS UTILISATIONS

(84) Designated Contracting States:
**AT BE CH CY DE DK ES FI FR GB GR IE IT LI LU MC NL PT SE TR**

(30) Priority: **21.09.2000 CA 2320664**

(43) Date of publication of application:
**18.06.2003 Bulletin 2003/25**

(73) Proprietor: **Novation Pharmaceuticals Inc.**
**Coquitlam,**
**British Columbia V3J 3B6 (CA)**

(72) Inventors:
- **KASTELIC, Tania**
  **Coquitlam, British Columbia V3J 3B6 (CA)**
- **CHENEVAL, Dominique**
  **Coquitlam, British Columbia V3J 3B6 (CA)**
- **LEUTWILER, Albert**
  **CH-3178 Bosingen (CH)**

(74) Representative: **Clayton-Hathway, Anthony Nicholas et al**
**Fry, Heath & Spence,LLP**
**The Gables,**
**Massetts Road**
**Horley,**
**Surrey RH6 7DQ (GB)**

(56) References cited:
**EP-A- 0 484 870**
**EP-A- 0 875 246**
**US-A- 5 767 147**

- **KUO Y-H ET AL: "SENSITIZED PHOTOOXIDATION OF 2-FURFURYL BENZOATE IN METHANOL" HETEROCYCLES, ELSEVIER SCIENCE PUBLISHERS B.V. AMSTERDAM, NL, vol. 24, no. 5, 1986, pages 1361-1366, XP001038437 ISSN: 0385-5414**
- **BEARD, A.R. ET AL.: "Synthesis o 2',3'-dideoxy-2',3'-alpha-methanocytidine" CARBOHYDRATE RESEARCH, vol. 205, 1990, page 87-91 XP001038501**
- **TSURUOKA T ET AL: "INHIBITION OF MOUSE TUMOR METASTASIS WITH NOJIRIMYCIN-RELATED COMPOUNDS" JOURNAL OF ANTIBIOTICS, JAPAN ANTIBIOTICS RESEARCH ASSOCIATION. TOKYO, JP, vol. 49, no. 2, February 1996 (1996-02), pages 155-161, XP001036852 ISSN: 0021-8820**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 318 991 B1

## Description

This invention relates to biologically active benzoic acid derivatives and to their use in the treatment and prophylaxis of disease. In particular the invention relates to a class of compounds which affect the stability of mRNA which contain one or more mRNA instability sequences.

Various benzoic acids have been found to manifest activities of therapeutic value. For example, in Japan Kokai 61-22046, 61-22047 and 61-76440, it was shown that benzoic acid derivatives represented by the following general formula:

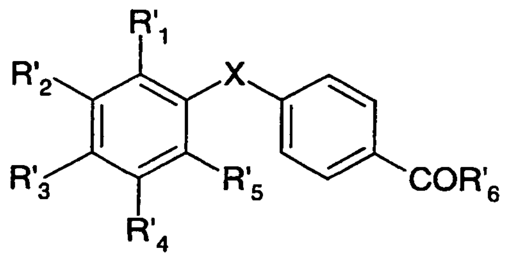

wherein R'1, R'2, R'3, R'4 and R'5 are the same or different, and each represents hydrogen or middle or lower-alkyl, with the proviso that all can not be hydrogen simultaneously, and wherein two neighboring substituents may be combined with each other to form a cyclo-alkyl ring having 5 to 6 carbon atoms, R'6 represents hydroxyl, lower-alkoxyl, or lower-alkylamino of the formula --NR'7 R'8, wherein R'7 and R'8 each represents hydrogen or lower-alkyl, and X' represents a group of the formula:

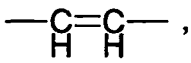 , 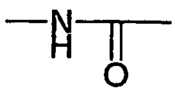 , 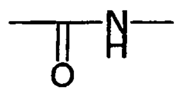 ,

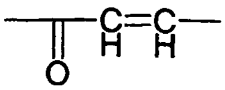 , or —N=N— ,

are capable of inducing the differentiation of malignant cells, especially leukemia cells, to morphologically and functionally mature cells which cannot proliferate further, and are accordingly pharmacologically valuable and useful for treatment of malignant proliferous or immune diseases such as cancer, rheumatism, or psoriasis, and in Japan Kokai 62-215581, there are also shown related compounds.

The literature also shows the activity and measurement of the activity of those compounds by the differentiation of human acute promyclocytic leukemia cells (HL-60).

U.S. Patent No. Re.36,477 also discloses benzoic acid derivatives which act as differentiation-inducing agents for neoplastic cells, especially leukemia cells, or as a therapeutic agent for psoriasis or immune and inflammatory diseases

U.S. Patent No. 5,849,796 discloses ortho-substituted benzoic acid derivatives that exhibit antiarrhythmic properties and act as inhibitors of the cellular $Na^+$ /$H^+$ antiporter. In addition, these compounds were shown to be useful as intermediates for the preparation of medicaments, in particular of inhibitors of the cellular $Na^+$ /$H^+$ antiporter.

This background information is provided for the purpose of making known information believed by the applicant to be of possible relevance to the present invention. No admission is necessarily intended, nor should be construed, that any of the preceding information constitutes prior art against the present invention. Publications referred to throughout the specification are hereby incorporated by reference in their entireties in this application.

An object of the present invention is to provide benzoic acid derivatives and uses thereof.

In accordance with one aspect of the present invention, there is provided a compound having structural formula (I):

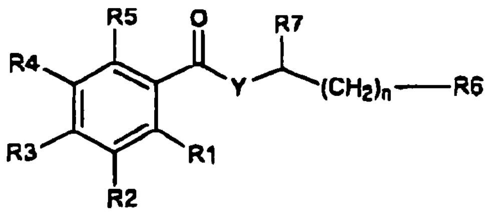

(I)

or stereoisomers thereof, or pharmaceutically acceptable salts or hydrates thereof or physiologically acceptable and hydrolysable esters thereof, wherein:

R1 is H, OH, halogen, ($C_1$-$C_4$) alkyl, ($C_1$-$C_4$) alkenyl, ($C_1$-$C_4$) alkoxy, or ($C_1$-$C_4$) alkyl-COO-;
R2 is H, OH, halogen, ($C_1$-$C_4$) alkyl, ($C_1$-$C_4$) alkenyl, ($C_1$-$C_4$) alkoxy, or ($C_1$-$C_4$) alkyl-COO-;
R3 is H, OH, halogen, ($C_1$-$C_4$) alkyl, ($C_1$-$C_4$) alkenyl, ($C_1$-$C_4$) alkoxy, or ($C_1$-$C_4$) alkyl-COO-;
R4 is H, OH, halogen, ($C_1$-$C_4$) alkyl, ($C_1$-$C_4$) alkenyl, ($C_1$-$C_4$) alkoxy, or ($C_1$-$C_4$) alkyl-COO-;
R5 is H, OH, halogen, ($C_1$-$C_4$) alkyl, ($C_1$-$C_4$) alkenyl, ($C_1$-$C_4$) alkoxy, or ($C_1$-$C_4$) alkyl-COO-; ($C_1$-$C_4$) alkenyl corresponds to up to $C_4$ alkenyl in the claims.
Y is O or $-NR^1$, wherein R' is H or ($C_1$-$C_4$) alkyl;
n is 0-8;
R6 is a 5 to 8 membered lactone or lactam ring;
R7 is H, ($C_1$-$C_4$) alkyl, ($C_1$-$C_4$) alkenyl, ($C_1$-$C_4$) alkoxy, phenyl or ($C_1$-$C_4$) alkyl-COO, wherein the lactam or lactone ring optionally comprises one or more C=C double bonds and the lactam or lactone ring is optionally substituted with one or more ($C_{1-}C_4$)alkyl or ($C_1$-$C_4$) alkenyl groups;

with the proviso that when R1 = R2 = R3 = R4 = R5 = R7 = H, and Y is O, and n is 0, then R6 is other than

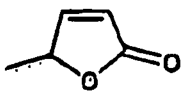 , 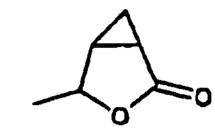 , 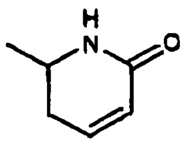 or 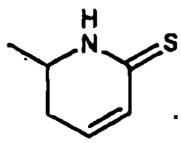 .

The compounds of the present invention are useful for the treatment of prevention of disorders with an etiology associated with or comprising excessive cytokine release, particularly IL-1β release, such as rheumatoid arthritis, osteoarthritis, septic shock, psoriasis, atherosclerosis; inflammatory bowel disease, Crohn's disease and asthma. The disclosure ofEP 0606044 A anticipates lactones for the treatment of diseases which are associated with recessive cytokine release.

The present invention also provides compounds for use in the preparation of a medicament for treatment of a cancer and/or malignant disease.

## BRIEF DESCRIPTION OF THE DRAWINGS

Figure 1 graphically depicts the percentage loss of viability of cancer cells following treatment with various concentrations of a benzoic acid derivative, in comparison to viability of control cells.

Figure 2 graphically depicts the relative growth of cancer cells following treatment with various concentrations of a benzoic acid derivative, in comparison to growth of control cells.

## DETAILED DESCRIPTION OF THE INVENTION

The terms and abbreviations used in the instant examples have their normal meanings unless otherwise designated. For example "°C" refers to degrees Celsius; "N" refers to normal or normality; "mmol" refers to millimole or millimoles; "g" refers to gram or grams; "ml" means milliliter or milliliters; "M" refers to molar or molarity; "*p*-" refers to

para, "MS" refers to mass spectrometry; "IR" refers to infrared spectroscopy; and "NMR" refers to nuclear magnetic resonance spectroscopy.

The present invention provides a compound of the formula I:

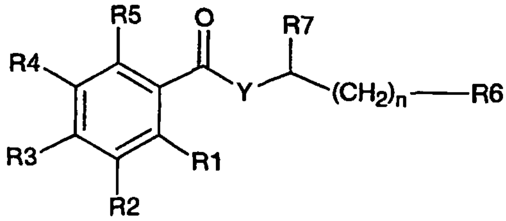

**(I)**

stereoisomers thereof, or phannaceutically acceptable salts or hydrates thereof or physiologically acceptable and hydrolysable esters thereof, wherein:

R1 is H, OH, halogen, $(C_1\text{-}C_4)$ alkyl, $(C_1\text{-}C_4)$ alkenyl, $(C_1\text{-}C_4)$ alkoxy, or $(C_{1\text{-}}C_4)$alkyl-COO-;
R2 is H, OH, halogen, $(C_1\text{-}C_4)$ alkyl, $(C_1\text{-}C_4)$ alkenyl, $(C_1\text{-}C_4)$alkoxy, or $(C_1\text{-}C_4)$ alkyl-COO-;
R3 is H, OH, halogen, $(C_1\text{-}C_4)$ alkyl, $(C_1\text{-}C_4)$ alkenyl, $(C_1\text{-}C_4)$alkoxy, or $(C_1\text{-}C_4)$ alkyl-COO-;
R4 is H, OH, halogen, $(C_1\text{-}C_4)$ alkyl, $(C_1\text{-}C_4)$ alkenyl, $(C_1\text{-}C_4)$alkoxy, or $(C_1\text{-}C_4)$ alkyl-COO-;
R5 is H, OH, halogen, $(C_1\text{-}C_4)$ alkyl, $(C_1\text{-}C_4)$ alkenyl, $(C_1\text{-}C_4)$alkoxy, or $(C_1\text{-}C_4)$ alkyl-COO-;
Y is O or -NR', wherein R' is H or $(C_1\text{-}C_4)$ alkyl;
n is 0-8;
R6 is a 5 to 8 membered lactone or lactam ring;
R7 is H, $(C_1\text{-}C_4)$ alkyl, $(C_1\text{-}C_4)$ alkenyl, $(C_1\text{-}C_4)$alkoxy, phenyl or $(C_1\text{-}C_4)$ alkyl-COO-,

wherein the lactam or lactone ring optionally comprises one or more C=C double bonds and the lactam or lactone ring is optionally substituted with one or more $(C_1\text{-}C_4)$alkyl or $(C_1\text{-}C_4)$ alkenyl groups. Halogen or halo as used herein refers to F, Cl, Br or I unless otherwise indicated, preferably Cl.

In one embodiment of the present invention a compound of formula (I) is provided in free form or in the form of a physiologically-hydrolysable and -acceptable ester, wherein:

R1 = H, OH, MeCOO- or Methoxy;
R2 = H;
R3 = H, OH, MeCOO- or Methoxy;
R4 = H, Methoxy or halogen;
R5 = H, OH, Methoxy or $(C_1\text{-}C_4)$ alkyl;
Y is O;
R6 is

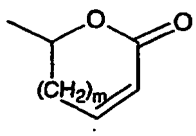

wherein, m is 0-4 ;
R7 = H, Methyl, Isopropyl.

In another embodiment of the present invention a compound of formula (I) is provided in free form or in the form of a physiologically-hydrolysable and -acceptable ester, wherein:

R1 is H, -OH, MeO- or MeCOO-;
R2 is H; $R_4$ is H or halogen;

R3 is H, -OH, MeO- or MeCOO-;
R4 is H or halogen;
R5 is -OH, MeO-, MeCOO-or Methyl.

In another embodiment of the present invention a compound of formula (I) is provided in free form or in the form of a physiologically-hydrolysable and -acceptable ester wherein:

R1 is H or MeO, $R_3$ is H, -OH or MeO-; $R_4$ is H and $R_5$ is Methoxy or Methyl.

Compounds of the present invention include, but are not limited to the following exemplary molecules:

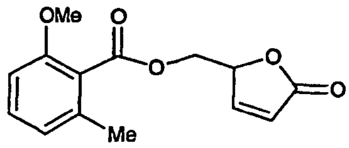 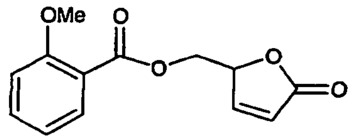 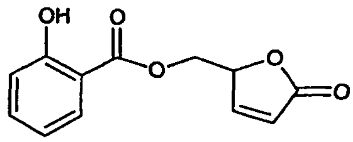

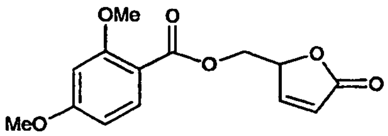 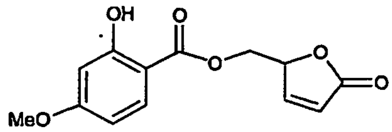 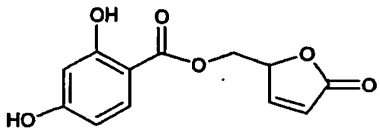

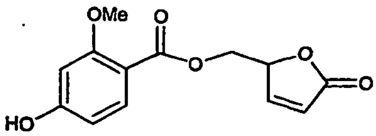 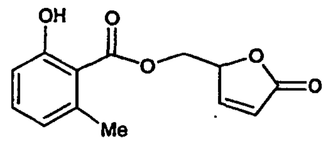

In another embodiment of the present invention Compounds of the present invention include, but are not limited to the following exemplary molecules:

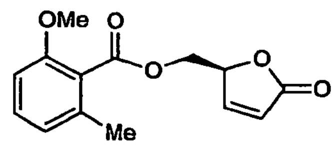 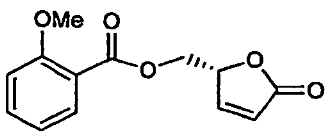 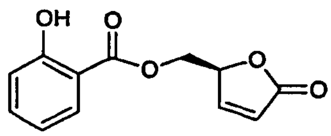

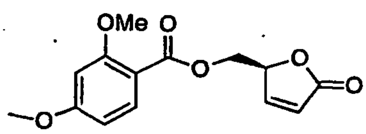 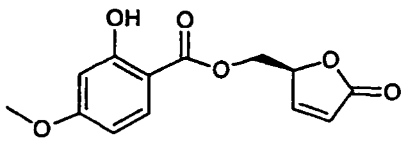 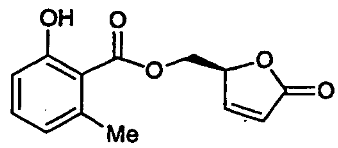

As noted supra, this invention includes the pharmaceutically acceptable salts of the compounds defined by Formula I. A compound of this invention can possess a sufficiently acidic, a sufficiently basic, or both functional groups, and accordingly react with any of a number of organic and inorganic bases, and inorganic and organic acids, to form a pharmaceutically acceptable salt.

This invention further encompasses the pharmaceutically acceptable solvates of the compounds of Formula I. Many of the Formula I compounds can combine with solvents such as water, methanol, ethanol and acetonitrile to form pharmaceutically acceptable solvates such as the corresponding hydrate, methanolate, ethanolate and acetonitrilate.

The compounds of the present invention may have asymmetric (chiral) centers. As a consequence of these chiral centers, the compounds of the present invention occur as racemates, mixtures of enantiomers and as individual enantiomers, as well as diastereomers and mixtures of diastereomers. All asymmetric forms, individual isomers (which may be prepared *via* stereospecific synthesis or by separation of a synthesized mixture using conventional methods) and combinations thereof, are within the scope of the present invention.

The prefixes "*R*" and "*S*" are used herein as commonly used in organic chemistry to denote the absolute configuration of a chiral center, according to the Cahn-Ingold-Prelog system. The stereochemical descriptor *R* (*rectus*) refers to that configuration of a chiral center with a clockwise relationship of groups tracing the path from highest to second-lowest priorities when viewed from the side opposite to that of the lowest priority group. The stereochemical descriptor *S* (*sinister*) refers to that configuration of a chiral center with a counterclockwise relationship of groups tracing the path from highest to second-lowest priority when viewed from the side opposite to the lowest priority group. The priority of groups is decided using sequence rules as described by Cahn *et al., Angew. Chem.,* 78, 413-447, 1966 and Prelog, V. and Helmchen, G.; *Angew. Chem. Int. Ed Eng.,* 21, 567-583, 1982).

In addition to the *R*,*S* system used to designate the absolute configuration of a chiral center, the older D-L system is also used in this document to denote relative configuration, especially with reference to amino acids and amino acid derivatives. In this system a Fischer projection of the compound is oriented so that carbon-1 of the parent chain is at the top. The prefix "D" is used to represent the relative configuration of the isomer in which the functional (determining) group is on the right side of the carbon atom at the chiral center and "L", that of the isomer in which it is on the left.

The compounds of the present invention have carbon-carbon double bonds and can, therefore form *cis*/*trans* isomers. The present invention envisages both mixtures of the isomers and the individual isolated/purified isomers.

The benzoic acid derivatives for use in the invention which comprise -OH substituents may also exist in the form of pharmaceutically acceptable, metabolically labile ester derivatives, and the use of such is included within the scope of the invention. Pharmaceutically acceptable esters are preferably prodrug ester derivatives, such being convertible by solvolysis or under physiological conditions to the free derivatives. Preferred pharmaceutically acceptable prodrug esters are those derived from a carboxylic acid, a carbonic acid monoester or a carbamic acid.

**Preparation of Compounds of Formula (I)**

According to another aspect, the present invention provides a process for the preparation of a compound of Formula I, or a pharmaceutically acceptable metabolically-labile ester or amide thereof, or a pharmaceutically acceptable salt thereof, which comprises:

a) reacting a compound of formula (II):

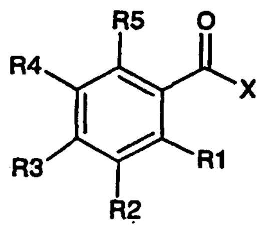

**(II)**

wherein:

R1 is H, OH, halogen, ($C_1$-$C_4$) alkyl, ($C_1$-$C_4$) alkenyl, ($C_1$-$C_4$) alkoxy, or ($C_1$-$C_4$)alkyl-COO-;
R2 is H, OH, halogen, ($C_1$-$C_4$) alkyl, ($C_1$-$C_4$) alkenyl, ($C_1$-$C_4$)alkoxy, or ($C_1$-$C_4$) alkyl-COO-;
R3 is H, OH, halogen, ($C_1$-$C_4$) alkyl, ($C_1$-$C_4$) alkenyl, ($C_1$-$C_4$)alkoxy, or ($C_1$-$C_4$) alkyl-COO-;
R4 is H, OH, halogen, ($C_1$-$C_4$) alkyl, ($C_1$-$C_4$) alkenyl, ($C_1$-$C_4$) alkoxy, or ($C_1$-$C_4$) alkyl-COO-; and
X is halogen;

with a compound of formula (III):

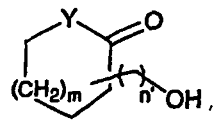

(III)

wherein:
n' is 1-9; and
m is 0-4;
compound (III) optionally contains one or more C=C double bonds and is substituted with one or more ($C_1$-$C_4$) alkyl or ($C_1$-$C_4$) alkenyl group; in the presence or absence of a base and an appropriate solvent. Non limiting examples of such solvents are dichloromethane and chloroform.

Compound of formula (II) can be prepared by reacting compound of formula (IV):

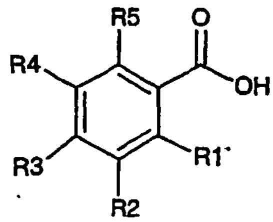

(IV)

wherein: R1-R5 is as defined above;
with acid halide of an organic or inorganic acid in the presence of an appropriate solvent known to a worker skilled in the relevant art. Non limiting examples of such acid halides are thionyl chloride, phosphorus trichloride, phosphorus tribromide, phosphorus pentachloride and oxalyl chloride and non limiting examples such solvents are DMF and THF; or

b) reacting compound of formula (IV) with compound of formula (II) in the presence of an acid, such as hydrochloric acid, sulfuric acid or aryl sulfonic acid. This reaction can be carried out under Azeotropic distillation conditions using benzene as a solvent; or

c) reacting an alkali or alkaline earth metal salt of compound of formula (IV):

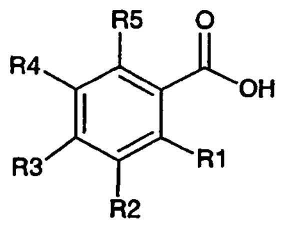

(IV)

wherein R1-R5 are as defined above;
with a compound of formula (V):

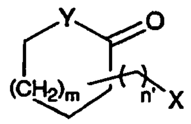

(V)

wherein;

X is F, Cl, Br or I;
n' is 1-9; and
m is 0-4; and compound of formula (V) may optionally contain one or more double bonds and is optionally substituted with one or more ($C_1$-$C_4$) alkyl or ($C_1$-$C_4$) alkenyl group;
in the presence of polar aprotic solvents or with crown ether catalysts. Non limiting examples of such polar aprotic solvents are acetone, DMFand DMSO.

Compounds of formula (IV) are either commercially available or may be prepared using standard procedures known to a person skilled in the art. For example: by hydrolyzing a compound of formula (VI):

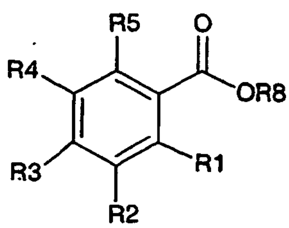

(VI)

wherein:

R1-R5 is as defined above;
R8 is ($C_1$-$C_4$) alkyl, aryl or arylaklyl.

The compounds of Formula (VI) are conveniently hydrolyzed in the presence of an acid, such as hydrochloric acid or sulfuric acid, or a base, such as an alkali metal hydroxide, for example sodium hydroxide. The hydrolysis conveniently performed in an aqueous solvent such as water and at a temperature in the range from 50 to 200 °C. Compounds of formula (VI) are either commercially available or may be prepared using standard procedures known to a person skilled in the art.

Compounds of formula (III) or (V) are either commercially available or may be prepared using standard procedures known to a person skilled in the art.

**Activity of the Benzoic Acid Derivatives**

The benzoic acid derivatives of the present invention exhibit various biochemical properties including one or more of the following: the ability to inhibit the growth of cancer cells, the ability to increase or restore sensitivity of a cancer cell to one or more anti-neoplastic/cytotoxic drugs; and the ability to induce mRNA degradation. Compounds of the present invention can be assayed to demonstrate the extent of their activities using standard techniques well known to workers skilled in the art. Exemplary testing methods are outlined herein and are not intended to limit the scope of the present invention.

*Anti-Cancer Activity*

As demonstrated herein certain benzoic acid derivatives according to the present invention can inhibit the growth of cancer cells. Compounds of the present invention may be tested for their activity as anti-cancer agents in cell or *in vivo* assays substantially as described herein or in variants of such assays using appropriate cell lines and conditions as would be readily appreciated by a worker skilled in the art.

Compounds of this invention may be assayed for anti-cancer activity by conventional methods, including for example, the methods described below.

1. *In Vitro* Methodology

Cytotoxicity may be measured using a standard methodology for adherent cell lines such as the microculture tetrazolium assay (MTT). Details of this assay have been published (Alley, M C et al, Cancer Research 48:589-601, 1988). Exponentially growing cultures of tumor cells such as the HT-29 colon carcinoma or LX-1 lung tumor are used to make microtiter plate cultures. Cells are seeded at 3000 cells per well in 96-well plates (in 150 μl or media), and grown overnight at 37°C. Test compounds are added, in 10-fold dilutions varying from $10^{-4}$M to $10^{-10}$M. Cells are then incubated for 72 hours. To determine the number of viable cells in each well, the MTT dye is added (50 μl or 3 mg/ml solution of 3-(4,5-dimethylthiazol-2-yl)-2,5-diphenyltetrazolium bromide in saline). This mixture is incubated at 37°C. for 5 hours, and then 50 μl of 25% SDS, pH2 is added to each well. After an overnight incubation, the absorbance of each well at 550 nm is read using an ELISA reader. The values for the mean ±SD of data from replicated wells are calculated, using the formula % T/C (% viable cells treated/control). 1 OD of treated cells OD of control cells × 100 + % T/C. The concentration of test compound which gives a T/C of 50% growth inhibition is designated as the $IC_{50}$ value.

An alternative method of evaluating anti-cancer activity of the compounds of the present invention involves determining the effect of a test compound on the viability of cancer cells. Exponentially growing cancer cells are treated with various concentrations of the test compound. Examples of cell lines that can be used in this method are MCF-7, MDA-MB-231, HCT116, KB 31, HCT-15, and DU 145 which are readily obtainable from ATCC, Bethesda, MD, U.S.A. The cells are further incubated for 24 h, 48h and 96 h and stained with a molecule that allows a distinction between viable and non-viable cells, for example YOPRO-1 as described in T. Idziorek et al., J Immunol Meth 185, 249 (1995). The percent viability is then compared to control cells. A decrease in viability (shown as an increase in % loss of viability in Figure 1) in the treated cells in comparison to the control cells is indicative of anti-cancer activity.

Another method of evaluating anti-cancer activity of the compounds of the present invention involves monitoring the effect of the compounds on cell cycle progression. Exponentially growing cancer cells are treated with various concentrations of the test compound. The cells were further incubated for 24 h, 48h and 96 h. Cells are then lysed and dsDNA is stained with YOPRO-1 as described in T. Idziorek et al., J Immunol Meth 185, 249 (1995). Fluorescence measurements are carried out essentially as described in V.L. Singer et al., Anal Biochem 249, 228 (1997) and values compared to control cells. A decrease in relative growth of treated cells in comparison to untreated cells is indicative of anti-cancer activity (see Figure 2).

In an exemplary method of evaluating the inhibitory effect of the compounds of the present invention on the growth of cancer cells, cells are treated with the test compound and gene expression levels are measured in comparison to untreated cells. For example, human breast cancer cells, MCF-7, are cultured at 5% $CO_2$ and 37°C in MEM containing 0.1 mM non-essential amino acids, 2 mM L-glutamine and 10% FBSe. Total RNA from cells approximately 50% confluent

is isolated using a Qiagen RNeasy Midi™ kit according to manufacturer's instructions. Total RNA is harvested at various times in the presence and absence of the benzoic acid derivative being evaluated. RNase protection analysis is performed using the RiboQuant™ Multi-Probe Ribonuclease Protection Assay System from Pharmingen utilizing hAPO-2c and custom human (bclx LIS, p53, GADD45, c-fos, bax, bcl-2, c-myc, L32 and GAPDH) templates, T7 RNA polymerase and [$\alpha$-$^{32}$P]UTP (as per manufacturer's instructions). Quantitation of the expression levels is performed using a Storm 860™ Molecular Dynamics Phosphoimager with ImageQuant™ software. Decreased expression of a proto-oncogene, for example bcl-2, in treated cells in comparison to untreated cells is indicative of anti-cancer activity.

2. *In Vivo* Methodology

Compounds of this invention may be further tested in pre-clinical assay for *in vivo* activity which is indicative of clinical utility. Such assays are conducted with nude mice into which tumor tissue, preferably of human origin, has been transplanted (xenografted), as is well known in this field. Test compounds are evaluated for their anti-tumor efficacy following administration to the xenograft-bearing mice.

For example, human breast tumors (MX-1) which have been grown in athymic nude mice are transplanted into new recipient mice, using tumor fragments which are about 50 mg in size. The day of transplantation is designated as day 0. Six to ten days later, mice are treated with the test compounds given as an intravenous injection or orally, in groups of 5-10 mice at each dose. Compounds are given every other day, for 3 weeks.

Tumor diameters and body weights are measured twice weekly. Tumor volumes were calculated using the diameters measured with Vernier calipers, and the formula

$$(\text{Length} \times \text{width}^2) \div 2 = \text{mm}^3 \text{ of tumor volume}$$

Mean tumor volumes are calculated for each treatment group, and T/C values determined for each group relative to the untreated control tumors.

*Increasing or Restoring Drug Sensitivity*

Certain benzoic acid derivatives according to the present invention can increase or restore the sensitivity of a cancer cell to one or more anti-neoplastic/cytotoxic drugs. A suitable cell-based assay for assessing the ability of a candidate compound to restore sensitivity of cancer cells to anti-neoplastic/cytotoxic drug substances *in vitro* is as follows. Cancer cell lines (CCL), e.g. from human small cell carcinoma of the lung, resistant to one or more cancer therapeutic drug substances (CTDS) selected from the group comprising Daunorubicin (DR); Vincristine (VC); Adriamycin (AM); Etoposide (ET); Tenoposide (TE); Colchicine (CC); and Taxol are developed in accordance with the methods described by Twentyman et al., Br. J. Cancer, 54, 253 (1986).

Sensitivity of resistant sub-lines (CCL-R) is compared with parental sensitive lines (CCL-S) by assaying inhibition of cell growth during continuous CTDS exposure, e.g. in the case of a DR-resistant line (CCL-DRR) by comparing growth of CCL-DRS and CCL-DRR lines in the presence of DR contained in the growth medium *ab initio.* For the purpose, cell proliferation is measured by cell counting using an electronic cell counter, counting being effected close to the termination of the exponential growth phase. CCL-R lines are selected for which the $IC_{80}$ (drug concentration, e.g. DR concentration, required to reduce final cell number to 20% of that for non-CTDS (e.g. DR) treated controls is >80 ×, preferably >100 ×, greater than that of the parental CCL-S lines.

Sensitivity of selected CCL-R lines to CTDS (e.g. DR) in the presence or absence of test benzoic acid derivatives is then performed, employing cell counting as a measure of proliferation as described above. For this purpose cells are cultured *ab initio* in the presence of varying concentrations of both CTDS and test benzoic acid derivatives. For screening, concentrations of the latter are chosen which do not themselves cause a significant reduction in proliferation. Appropriate concentrations are established by culturing CCL-S and CCL-R in the presence of varying concentrations of benzoic acid derivatives in the absence of CTDS. Benzoic acid derivatives are routinely tested at concentrations of from 0.01 to 50 $\mu$g/ml, in particular 0.1 to 10.0 $\mu$g/ml, e.g. at concentrations of 0.01, 0.02, 0.05, 0.1, 0.2, 0.5, 1.0, 2.0, 5.0, 10.0 and 50 □g/ml. The ratio of CTDS (e.g. DR) required to inhibit cell proliferation by 50% in the absence of test benzoic acid derivatives ($IC_{50}$-CS) compared with that obtained in the presence of test benzoic acid derivatives ($IC_{50}$+CS) is taken as a measure of increased sensitivity of the CCL-R line to CTDS which has been induced by the benzoic acid derivatives.

Stability of the CCL-R line used is ensured by cross checking its sensitivity to CTDS with that previously established.

Additional procedures for assessing utility in restoring sensitivity of cancer cells to anti-neoplastic/cytotoxic, drug substances, including *in vivo* procedures are described in EP 0296122 B, the relevant disclosures of which are

incorporated by reference in the teaching of the present application.

*Induction of mRNA Degradation*

Recently, it has become increasingly apparent that the regulation of RNA half-life plays a critical role in the tight control of gene expression and that mRNA degradation is a highly controlled process. RNA instability allows for rapid up- or down-regulation of mRNA transcript levels upon changes in transcription rates. A number of critical cellular factors, e.g. transcription factors such as c-myc, or gene products which are involved in the host immune response such as cytokines, are required to be present only transiently to perform their normal functions. Transient stabilization of the mRNAs which code for these factors permits accumulation and translation of these messages to express the desired cellular factors when required; whereas, under non-stabilized, normal conditions the rapid turnover rates of these mRNAs effectively limit and "switch off" expression of the cellular factors. However, abnormal regulation of mRNA stabilization can lead to unwanted build up of cellular factors leading to undesirable cell transformation, e.g. tumor formation, or inappropriate and tissue damaging inflammatory responses.

Although the mechanisms which control mRNA stability are far from understood, sequence regions have been identified in a number of mRNAs, which appear to confer instability on the mRNAs which contain them. These sequence regions are referred to herein as "mRNA instability sequences". For example, typical mRNA instability sequences are the AREs (AU rich elements), which are found in the 3'UTR (untranslated region) of certain genes including a number of immediate early genes and genes coding for inflammatory cytokines, e.g. IL-1β and TNFα.

mRNA instability sequences have been identified in the UTRs, in particular the 3' UTRs, of a large number of transiently expressed genes including genes for cytokines, chemokines, nuclear transcription factors, protooncogenes, immediate early genes, cell cycle controlling genes, oxygenases, and genes involved in and controlling of apoptosis. The natural RNA sequences which comprise the mRNA instability sequences are alternatively referred to as adenylate/uridylate (AU)-rich elements, or AREs. Transiently expressed genes which contain mRNA instability sequences include, for example, the genes coding for *GM-CSF, c-fos, c-myc, c-jun, krox-20, nur-77, zif268, IFN*-β*, uPA, IL-1, IL-3, TNF-*α*, MCP1, syn1,* $\beta_2$*-AR, E- selectin, VCAM- 1, ICAM- 1, P- glycoproteins (MDR), MRPs, P*γ*h1 (pf mdr), COX II, metalloproteinases (MMPs), bcl- 2* and *MIP-2*α.

The following publications include extensive discussion of mRNA instability sequences and AREs, the sequences motifs which they contain and (minimum) sequence requirements for mRNA destabilization, as well as identifying a number of mRNA instability sequences and the genes which contain them:

Shaw & Kamen, Cell, Vol. 46, 659-667, Aug. 29 1986 (GM-CSF);
Shyu et al., Genes & Development, 5:221-231 (1991) (c-*fos*);
Sachs, Cell, Vol. 74, 413-421, Aug. 13 1993 (Review. "Messenger RNA Degradation in Eukaryotes");
Chen et al., Mol. Cell. Biol., Jan 1994, p 416-426 (c-*fos*);
Akashi et al., Blood, Vol. 83, No. 11, (June 1), 1994: pp 3182-3187 (GM-CSF etc.); Nanbu et al., Mol. Cell. Biol., July 1994, p. 4920-4920 (Upa);
Stoecklin et al., J. Biol. Chem., Vol. 269, No. 46, Nov. 18 1994, pp 28591-28597 (IL-3);
Lagnado et al., Mol. Cell. Biol., Dec. 1994, p. 7984-7995 (general);
Zhang et al., Mol. Cell. Biol., Apr. 1995, p. 2231-2244 (yeast);
Zubiaga et al., Mol. Cell. Biol., Apr. 1995, p. 2219-2230 (general);
Winstall et al., Mol. Cell. Biol., July 1995, p. 3796-3804 (c-*fos*, GM-CSF);
Chen et al., Mol. Cell. Biol., Oct. 1995, p. 5777-5788 (c-*fos*, GM-CSF);
Chen et al., TIBS 20 Nov. 1995, 465-470 (review);
Levy et al., J. Biol. Chem., Vol. 271, No. , Feb. 2 1996, pp. 2746-2753 (VEGF); Kastelic et al., Cytokine, Vol. 8, No. 10 (Oct.), 1996: pp751-761;
Crawford et al., J. Biol. Chem., Vol. 272, No. 34, Aug. 1997, pp. 21120-21127 (TNF-α);
Xu et al., Mol. Cell. Biol., Aug. 1997, Vol. 18, No. 8, p. 4611-4621 (general);
Danner et al., J. Biol. Chem., Vol.273, No. 6, Feb. 6 1998, pp. 3223-3229 (human $\beta_{2}$-adrenergic receptor);
Lewis et al., J. Biol. Chem., Vol. 273, No. 22, May 29 1998, pp. 13781-13786 (TNF-α).

As described in the above publications, mRNA instability sequences often contain one or more copies of sequence motifs, e.g. selected from: AUUUA, UAUUUAU, UUAUUUA(U/A)(U/A), and AUUUAUUUA. Such sequence motifs are typically in genes between the stop codon and the poly A signal and may associated with appropriate flanking sequences and may interact in combination with other sequences, e.g. present in the 5' UTR and e.g. with instability motifs present in the coding region.

As demonstrated herein certain of the benzoic acid derivatives of the present invention can act as inducers of degradation of mRNAs which contain mRNA instability sequences. The activity of compounds for use in the invention

as inducers of mRNA degradation may be demonstrated using assays known to workers skilled in the art, for example by means of a reporter gene assay as described herein, or as described in more detail in International patent application number PCT/CA99/01235.

One method for identifying a the ability of a compound of the present invention to induce mRNA degradation, comprises i) contacting the compound with a DNA expression system which in the absence of the compound is capable of expressing a protein having a detectable signal, wherein the mRNA which codes for the protein and which is transcribed from the expression system comprises at least one copy of a mRNA instability sequence; ii) measuring the detectable signal in the presence of the test compound and comparing the result obtained with a control. A related method can be used to compare compounds which induce mRNA degradation. This method comprises separately contacting the compounds with a DNA expression system which in the absence of the compounds is capable of expressing a protein having a detectable signal, wherein the mRNA which codes for the protein and which is transcribed from the expression system comprises at least one copy of a mRNA instability sequence, measuring the detectable signal in the presence of each test compound and comparing the signals obtained.

The DNA expression system used in the above described methods typically comprises a gene coding for expression of the protein having a detectable signal, wherein the gene comprises DNA coding for the amino acid sequence of the protein together with associated 5' and 3'UTR sequences comprising appropriate expression control elements including promoter and/or enhancer regions, and characteristically DNA corresponding to at least one copy of a mRNA instability sequence. An exemplary DNA expression system that can be used in the above methods is a cell based expression system, conveniently in the form of a suitably transformed cell line, optionally a stably transformed cell line. The host cell is typically a eukaryotic host cell, in particular an animal host cell such as a mammalian host cell.

The host cell may be of the same general cell type as the cells which express the protein which is coded for by the mRNA which it is desired to destabilize. Thus for instance, if the assay is to be used for the characterization of compounds of the present invention to demonstrate their ability to destabilize the mRNA coding for a cytokine, the host cell used is preferably a cell or cell line which is of the same or similar cell type to the cells which normally produce the cytokine in question. For example, monocyte or monocyte-like cell lines may be used as host cells for assaying for compounds which destabilize cytokine, e. g. IL-1 ss, mRNA. Preferred cell lines for oncogene and other cancer related gene mRNA instability assays are, e.g. Colon 205, KB 31, KB 8511, DU145, HCT116, MCF7, MCF7/ADR, MDA-MB-231, MDA-MB-435-and MDA-MB-435/TO.

The gene coding for expression of the protein having a detectable signal may encode a protein that may itself comprise the detectable signal. For instance, the protein may comprise a fluorescent protein, e.g. green fluorescent protein.

Alternatively, the protein is such that it is capable of reacting with an appropriate substrate or other substance to give a detectable signal. Conveniently the protein encoded by the mRNA is an enzyme or enzymatically active fragment of an enzyme. Examples of suitable enzymes include horseradish peroxidase (HRP), chloramphenicol acetyltransferase (CAT), alkaline phosphatase (AP), secreted alkaline phosphatase (SEAP), β-galactosidase, or luciferase. Methods for detecting and quantifying such enzymes are well-known, using appropriate substrates and measurements. It will be appreciated, however, that any suitable detectable protein and measurement procedure may be used the methods described herein.

Alternative methods for measuring the ability of test molecules to induce mRNA degradation are known to workers skilled in the art and may be used to measure the activity of the benzoic acid derivatives of the present invention.

_Anti-Inflammatory Activity_

As demonstrated herein certain of the benzoic acid derivatives of the present invention exhibit anti-inflammatory activity, for example due to their ability to inhibit release of cytokines or act as antagonists of cytokine activity.

1. *In Vitro* Methodology

One exemplary method of measuring the ability of the benzoic acid derivatives of the present invention to inhibit cytokine release is performed by monitoring the release of cytokines from cells that have been stimulated to release cytokines either in the presence or the absence of the test compound. Lipopolysaccharide (LPS) is one type of stimulant that can be used to induce cytokine release although a worker skilled in the art would readily appreciate that other stimulants can be used in this assay. Following treatment of the cells in culture by the stimulant, with and without the test compound, the cells are incubated for an appropriate period of time and then the culture medium and cell lysates are collected. Assays are then performed using the isolated media and lysates to determine the amount of cytokine (e.g. IL-1β (medium and lysate), IL-6 (medium), TNF-α (medium) and $PGE_2$ (medium and lysate)) present in each sample. In order to ensure that the compounds are also non-toxic to the cells, lactate dehydrogenase (LDH) activity (medium and lysate) and DNA (lysates) are also analyzed. IL-1β, IL-6 and TNF-α assays can be determined using commercially

available ELISA kits (Cistron), $PGE_2$ can be measured using a standard RIA and DNA fluorimetrically using DAPI (4', 6-diamidino-2-phenylindole·2HCl). It should be recognized that this test is not limited by the assay(s) used to quantify the cytokines, LDH release or DNA since multiple assays are well known to workers skilled in the art and the assays listed herein are not intended to limit the scope of the present invention. A reduction in the amount of cytokine in the medium from the treated cells in comparison to the medium from the untreated cells ins indicative of the ability of the test compound to inhibit cytokine release. Furthermore, the compounds are considered to be non-toxic if LDH release is unchanged in the treated versus the untreated cells.

Another exemplary method of measuring the ability of the benzoic acid derivatives of the present invention to inhibit cytokine release is performed by comparing cytokine release from cells stimulated in culture in the presence and absence of the test compound. In an example of such a method mononuclear cells are obtained and cultivated on tissue culture dishes with the test compound at various concentrations [Schnyder et al., Agents & Actions, **30**, 350-362 (1990)]. The non-adherent lymphocytes are removed after 4 hrs by washing several times. Fresh medium, test compound and stimulant, for example lipopolysaccharide (LPS), are added and the monocytes incubated for a further day.

Purified IL-1, recombinant human IL-1-β (rhIL-1) or conditioned media collected from stimulated human monocytes, mouse macrophages or mouse cell line $P388D_1$, cause characteristic changes in the secretory pattern of chondrocytes. In particular, a latent metalloproteinase is induced, whilst secretion of plasminogen activator is reduced. The property of the metalloproteinase or stromelysin has been described in detail [Chin et al., J. Biol. Chem. **260,** 12367-12376 (1985)], as has that of the plasminogen activator [Schnyder et al., Anal. Biochem. **200**, 156-162 (1992)]. In performing the assay the pooled culture media are diluted 1:10 with fresh medium and added to confluent rabbit chondrocytes. Metalloproteinase activity in the chondrocyte culture medium is assayed after a further 2 days using standard techniques known to workers skilled in the art. One example of a technique for measuring metalloproteinase activity is provided in Example 6 of the present disclosure. Compounds having the ability to reduce cytokine release will decrease induction of metalloprotease activity in the chondrocytes that are pooled with the culture media from treated cells in comparison to those pooled with the culture media from untreated cells.

In order to determine the antagonistic effects of the benzoic acid derivatives of the present invention a cytokine is added to cultured cells that are known to respond to the cytokine and their response is monitored in the presence and the absence of the test compound. Example 7 of the present disclosure demonstrates one example of such a test in which chondrocytes are used. As described herein, chondrocytes will respond to IL-1 by an increase in metalloproteinase activity. A test compound is demonstrated to have an antagonistic effect if the metalloproteinase activity is decreased in the presence of the test compound in comparison to the activity in its absence.

2. *In Vivo* Methodology

One art recognized animal model of an inflammatory response is LPS-induced fever in rats. An LPS-suspension is injected in rats. At a certain time interval following injection the body temperature of the rat is measured and following the next interval of time the test compound is administered. At the end of the third time interval the body temperature is measured again. The temperature increment shown by the untreated controls can be taken as 100 % and that in the treated group can be expressed as a percentage of this value. The $ED_{50}$ is the dose causing a 50 % inhibition of the temperature increase determined in the control rats. Compounds of the present invention typically have an $ED_{50}$ in this assay of from about 0.1 to about 1 mg/kg.

An art recognized animal model of inflammation is carrageenan-induced paw edema in the rat. The test compound is administered to the rats one hour prior to the carrageenan injection. Carrageenan is given by subplantar injection into one hind paw. The swelling of the paw is measured. A control reading is taken immediately after the injection, and the swelling is measured after 3 and 5 hrs. The mean value of the 3- and 5-hour reading is taken after deduction of the control reading, and the values obtained from the treated animals are expressed as a percentage of the value obtained from non-treated controls. The $ED_{50}$ is the dose causing a 50 % inhibition of the carrageenan-induced swelling after 3 hr. Compounds of the invention typically have $ED_{50}$'s in this assay of from about 0.5 to about 1 mg/kg.

It should be readily appreciated to a worker skilled in the art that the tests described herein are examples of standard tests that can be used to measure the activity of the compounds of the present invention. A wide variety of tests for measuring anti-cancer or anti-neoplastic activity, ability to increase or restore drug sensitivity, ability to induce mRNA degradation and anti-inlfammatory activity are well known and can be used to demonstrate the characteristics of the benzoic acid derivatives of the present invention.

**Use of the Benzoic Acid Derivatives**

In one embodiment of the present invention the benzoic acid derivatives are used in the treatment of patients having pathological conditions associated with abnormal cell proliferation, such as cancer. The pathological conditions include the abnormal cell proliferation of malignant cells of various tissues and/or organs, comprising, with no limitation

being implied, muscle, bone or connective tissues, the skin, brain, lungs, sex organs, the lymphatic or renal systems, mammary or blood cells, liver, the digestive tract, pancreas and thyroid or adrenal glands. These pathological conditions can also include solid tumors, cancers of the ovary, breast, brain, prostate, colon, stomach, kidney or testicles, Kaposi's sarcoma, cholangioma, chorioma, neuroblastoma, Wilms' tumor, Hodgkin's disease, melanomas, multiple myelomas, lymphatic leukemias and acute or chronic granulocytic lymphomas.

In an alternative embodiment of the present invention the benzoic acid derivatives are used to inhibit the release of cytokines and/or as functional antagonists of cytokines. In a specific embodiment the benzoic acid derivatives are used to inhibit the release of IL-1, IL-6 and TNF-$\alpha$ and/or as functional antagonists of IL-1.

A related embodiment of the present invention involves the use of the benzoic acid in the treatment of disorders with an aetiology associated with or comprising excessive cytokine release, particularly IL-1$\beta$ release, e.g. in a wide variety of inflammatory states and diseases such as rheumatoid arthritis, osteoarthritis, septic shock, psoriasis, atherosclerosis, inflammatory bowel disease, Crohn's disease and asthma.

In another embodiment the compounds of the present invention may be used in the prophylaxis or treatment of diseases and medical conditions in general having an etiology associated with the increased or prolonged stability of mRNAs which contain one or more mRNA instability sequences, and which on prolonged or inappropriate expression typically give rise to undesirable effects, e.g. cancer cell growth or an unwanted inflammatory response.

Compounds of the present invention may be used in connection with diseases and medical conditions associated with any of the genes mentioned above or described in the listed publications, which comprise mRNA instability sequences.

Examples of diseases and medical conditions which may be treated or prevented by use of the present invention include: cancers (e.g. of the colon, breast, lung etc.), acute and chronic inflammation, autoimmune diseases, respiratory diseases, infectious diseases and transplant rejection, as well as, neuronal inflammation, Alzheimer's disease and cardiovascular diseases.

In a related embodiment the compounds of the present invention which induce degradation of mRNA that contains one or more mRNA instability sequences are provided for use as pharmaceuticals, e.g. for use in the prophylaxis or treatment of diseases and medical conditions in general having an etiology associated with the increased or prolonged stability of mRNAs which contain one or more mRNA instability sequences, and which on prolonged or inappropriate expression typically give rise to undesirable effects, e.g. cancer cell growth or an unwanted inflammatory response.

In view of their activity as inducers of degradation of mRNAs which contain mRNA instability sequences, the compounds of this invention are useful for the prophylaxis and treatment of cancers and malignant diseases which involve inappropriate build-up and expression of mRNAs, which contain mRNA instability sequences, and which code for proteins involved in the initiation, progression or persistence of cancer or malignant disease. Examples of cancer related genes, with mRNAs which contain mRNA destabilising sequences, include various oncogenes and transcription factors, e.g. c-myc, c-fos, Spl, bcl-2 and similar genes. The inappropriate or prolonged expression of such oncogenes is implicated in the initiation of certain forms of cancer, such as colon cancer, breast cancer, lung cancer etc.. Further examples of cancer related genes, with mRNAs which contain mRNA instability sequences are genes for metalloproteinase enzymes, e.g. MMP-1, MMP-2, collagenases etc., involved in tissue remodelling required for tumour growth and metastasis invasion; cell cycle related genes such as p45/SKIP2 etc. and multidrug resistance genes, e.g. mdr-1, MRPs, etc. involved in the intrinsic or acquired multidrug resistance of some cancer cells.

Treatment with compounds of this invention advantageously leads to degradation of the mRNAs of such genes, resulting in the down-regulation or "switching off" of gene expression. Thus for example, they may be use for treatment and prevention of oncogene mediated cancers and malignant diseases, to treat or prevent tumour growth and metastasis invasion in general, and to prevent or reverse multidrug resistance and thereby facilitate cancer and tumor treatment with conventional, e.g. cytotoxic, anti-cancer agents.

Characteristically when the benzoic acid derivatives are use to prevent or reverse multidrug resistance of tumor and other malignant cells, they are used in combination with cytostatic or cytotoxic agents.

The benzoic acid derivatives according to the present invention can be used either alone or in combination with other pharmacologically active compounds, for example together with inhibitors of the enzymes of polyamine synthesis, inhibitors of protein kinase C, inhibitors of other tyrosine kinases, cytokines, negative growth regulators, for example TGF-$\beta$ or IFN-$\beta$, aromatase inhibitors, antioestrogens and/or cytostatic agents.

### Compositions Comprising the Benzoic Acid Derivatives

In one embodiment of the present invention there is provided compositions comprising one or more benzoic acid derivative of Formula I and one or more non-toxic pharmaceutically acceptable carriers and/or diluents and/or adjuvants and, if desired, other active ingredients. As indicated above such compositions are used in the treatment of various conditions in mammals, including humans.

The compounds of the invention may be administered by any conventional route, in particular nasally, enterally,

preferably orally, e.g. in the form of tablets or capsules, or parenterally e.g. in the form of injectable solutions or suspensions or in a suppository form. Unit dosage forms contain, for example of from about 2 mg to 1 g of the compound of the invention. The invention also provides a pharmaceutical composition comprising an effective amount of the compounds in association with a pharmaceutically acceptable diluent or carrier. Such compositions may be manufactured in conventional manner.

Suitable pharmaceutical compositions comprising the compounds of the present invention as an active ingredient and that can be used especially in the treatment of the diseases mentioned above include compositions for enteral, such as nasal, buccal, rectal or especially oral, administration and parenteral, such as intravenous, intramuscular or subcutaneous, administration to warm-blooded animals, especially human beings. The compositions comprise the active ingredient on its own or preferably together with a pharmaceutically acceptable carrier. The dosage of the active ingredient depends on the disease to be treated, and on species, age, weight and individual condition, individual pharmacokinetic conditions, and the mode of administration. The dosage can be readily determined by a worker skilled in the art using standard methods in light of the above considerations.

The pharmaceutical compositions may comprise from approximately 1% to approximately 95% active ingredient, forms of administration in single dose form preferably comprising from approximately 20% to approximately 90% active ingredient and forms of administration that are not in single dose form preferably comprising from approximately 5% to approximately 20% active ingredient. Unit dose forms are, for example, dragées, tablets, ampoules, vials, suppositories or capsules. Other forms of administration are, for example, ointments, creams, pastes, foams, tinctures, lipsticks, drops, sprays, dispersions, etc. Examples are capsules comprising from approximately 0.05 g to approximately 1.0 g of the active ingredient.

The pharmaceutical compositions are prepared in a manner known *per se*, for example by means of conventional mixing, granulating, confectioning, dissolving or lyophilizing procedures.

Solutions of the active ingredient, and also suspensions or dispersions, especially isotonic aqueous solutions, dispersions or suspensions, are preferably used, it being possible, for example in the case of lyophilized compositions that contain the active ingredient alone or together with a carrier, for example mannitol, for such solutions, suspensions or dispersions to be made up prior to use. The pharmaceutical compositions may be sterilized and/or may comprise excipients, for example preservatives, stabilizers, wetting agents and/or emulsifiers, solubilizers, salts for regulating the osmotic pressure and/or buffers, and are prepared in a manner known *per se*, for example by means of conventional dissolving or lyophilizing procedures. The solutions or suspensions may comprise viscosity-increasing substances, such as sodium carboxymethylcellulose, dextran, polyvinylpyrrolidone or gelatin.

Suspensions in oil comprise as the oil component the vegetable, synthetic or semisynthetic oils customary for injection purposes. There may be mentioned as such especially liquid fatty acid esters that contain as acid component a long-chained fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid, or corresponding unsaturated acids, for example oleic acid, elaidic acid, erucic acid, brassidic acid or linoleic acid, if desired with the addition of antioxidants, for example vitamin E, β-carotene or 3,5-di-tert-butyl-4-hydroxytoluene. The alcohol component of those fatty acid esters has a maximum of 6 carbon atoms and is a mono- or poly-hydric, for example a mono-, di-or tri-hydric, alcohol, for example methanol, ethanol, propanol, butanol or pentanol or the isomers thereof, but especially glycol and glycerol. The following examples of fatty acid esters are therefore to be mentioned: ethyl oleate, isopropyl myristate, isopropyl palmitate, "Labrafil M 2375" (polyoxyethylene glycerol trioleate, Gattefossé, Paris), "Labrafil M 1944 CS" (unsaturated polyglycolised glycerides prepared by alcoholysis of apricot kernel oil and consisting of glycerides and polyethylene glycol ester; Gattefossé, France), "Labrasol" (saturated polyglyeolised glycerides prepared by alcoholysis of TCM and consisting of glycerides and polyethylene glycol ester; Gattefossé, France) and/or "Miglyol 812" (triglyceride of saturated fatty acids with a chain length of $C_8$ to $C_{12}$, Hüls AG, Germany), but especially vegetable oils, such as cottonseed oil, almond oil, olive oil, castor oil, sesame oil, soybean oil and more especially groundnut oil.

The injection compositions are prepared in customary manner under sterile conditions; the same applies also to introducing the compositions into, for example, ampoules or vials and to sealing the containers.

Pharmaceutical compositions for oral administration can be obtained, for example, by combining the active ingredient with one or more solid carriers, if desired granulating a resulting mixture, and processing the mixture or granules, if desired, and if necessary by the addition of additional excipients, to form tablets or dragée cores.

Suitable carriers are especially fillers, such as sugars, for example lactose, saccharose, mannitol or sorbitol, cellulose preparations and/or calcium phosphates, for example tricalcium phosphate or calcium hydrogen phosphate, and also binders, such as starches, for example corn, wheat, rice or potato starch, methylcellulose, hydroxypropylmethylcellulose, sodium carboxymethylcellulose and/or polyvinylpyrrolidone, and/or, if desired, disintegrators, such as the above-mentioned starches, also carboxymethyl starch, crosslinked polyvinylpyrrolidone, or alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Dragée cores can be provided with suitable, optionally enteric, coatings, there being used *inter alia* concentrated sugar solutions which may contain gum arabic, talc, polyvinylpyrrolidone, polyethylene glycol and/or titanium dioxide, or coating solutions in suitable organic solvents or solvent mixtures, or, for the production of enteric coatings, solutions of suitable cellulose preparations, such as acetylcellulose phthalate or hydroxypropylmethylcellulose phthalate. Colorings or pigments may be added to the tablets or dragée coatings, for example for identification purposes or to indicate different doses of active ingredient.

Orally administrable pharmaceutical compositions also include dry-filled capsules consisting of gelatin, and also soft, sealed capsules consisting of gelatin and a plasticizer, such as glycerol or sorbitol. The dry-filled capsules may contain the active ingredient in the form of granules, for example in admixture with fillers, such as corn starch, binders and/or glidants, such as talc or magnesium stearate, and optionally stabilisers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilisers and detergents, for example of the polyoxyethylene sorbitan fatty acid ester type, may also be added.

Other oral forms of administration are, for example, syrups prepared in customary manner which comprise the active ingredient, for example, in suspended form and in a concentration of about 5 % to 20 %, preferably about 10 %, or in a similar concentration that provides a suitable single dose, for example, when administered in measures of 5 or 10 ml. Also suitable are, for example, powdered or liquid concentrates for the preparation of shakes, for example in milk. Such concentrates may also be packaged in single dose quantities.

Suitable rectally administrable pharmaceutical compositions are, for example, suppositories that consist of a combination of the active ingredient and a suppository base. Suitable suppository bases are, for example, natural or synthetic triglycerides, paraffin hydrocarbons, polyethylene glycols or higher alkanols.

For parenteral administration there are suitable especially aqueous solutions of an active ingredient in water-soluble form, for example in the form of a water-soluble salt, or aqueous injection suspensions that contain viscosity-increasing substances, for example sodium carboxymethylcellulose, sorbitol and/or dextran, and, if desired, stabilisers. The active ingredient, optionally together with excipients, can also be in the form of a lyophilisate and can be made into a solution prior to parenteral administration by the addition of suitable solvents.

The compounds for use in the invention can be administered, prophylactically or therapeutically, as such or in the form of pharmaceutical compositions, preferably in an amount effective against the said diseases, to a warm-blooded animal, for example a human being, requiring such treatment, the compounds being used especially in the form of pharmaceutical compositions. In such treatment an individual of about 70 kg body weight will be administered a daily dose of from approximately 0.1 g to approximately 5 g, preferably from approximately 0.5 g to approximately 2 g, of a compound.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the pharmaceutical compositions of the present invention may be employed in conjunction with other therapeutic compounds.

To gain a better understanding of the invention described herein, the following examples are set forth. It should be understood that these examples are for illustrative purposes only. Therefore, they should not limit the scope of this invention in any way.

**EXAMPLES**

**EXAMPLE 1: Preparation of the Compound of Formula D and Derivatives**

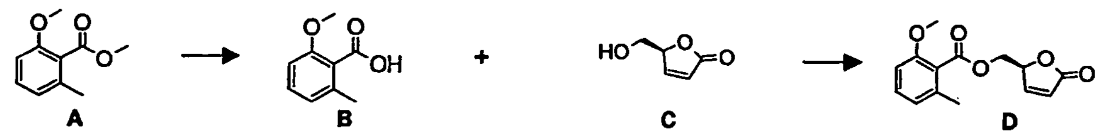

A B C D

*Compound B*

To a solution of 46.7 g compound A (which has been synthesized following the literature procedure in Synthesis 1980, 814) in 800 ml of methanol were added 400 ml of 2N NaOH. This mixture was then refluxed for 24 hrs. Methanol was evaporated off and the remaining aqueous liquor was extracted with diethylether. The aqueous phase was cooled and acidified with conc. HCl. The precipitation was filtered off, washed well with water and dried at 80°C under vacuum to give 31.8 g of a beige solid. M.p. 138-140°C.

*Compound D*

To a solution of 1.55 g compound B (prepared above) in 42 ml of THF was added 4.1 ml of oxalic acid chloride dropwise at 20°C. The solution was stirred at room temperature (RT) for 2 hrs and then the solvents were removed on the rotary evaporator. The residue was dissolved with 40 ml methylene chloride and 0.8 ml pyridine. A solution of 1 g compound C (which was synthesized following the procedure in J. Chem. Research (3) 1987, 369) in 10 ml methylene chloride was added dropwise. The mixture was stirred under argon at RT for 17 hrs and then extracted with sat. aq. $NaHCO_3$ solution. The organic phase was dried and evaporated to leave 1.97 g of a brown oil which was purified on a silica gel column with hexane : ethyl acetate = 2 : 1 as solvent giving 0.9 g compound D as colourless crystals. M.p. 74-78°C.

D: NMR (in $CDCl_3$): 2.28 ppm (s, 3H); 3.8 ppm (s, 3H); 4.59 ppm (d, 2H); 5.32 ppm (m, 1H); 6.21 ppm (dd, 1H); 6.7-6.85 ppm (m, 2H); 7.23 ppm (t, 1H); 7.51 ppm (dd, 1H).

**EXAMPLE 2: Preparation of the Compound of Formula F and Derivatives**

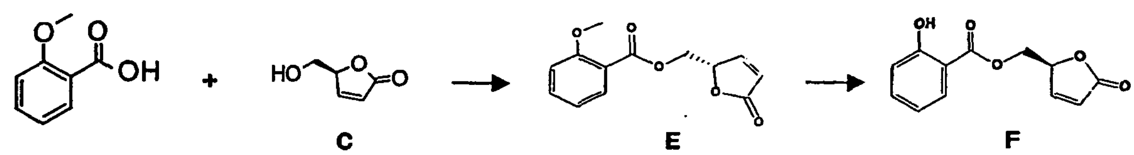

C E F

*Compound E*

To a solution of 3.6 g of 2-Methoxy-benzoic acid were added dropwise 10.1 ml of oxalic acid chloride at 20°C, plus 1 drop of DMF. The solution was stirred at RT for 3 hrs. The pale yellow solution was evaporated down and re-dissolved in 100 ml of dichloromethane. To this solution was added a solution of 2.5 g compound C (prepared above) in 25 ml of dichloromethane. This mixture was stirred at RT for 24 hrs, then washed with water, dried with $Na_2SO_4$, and evaporated down to give a dark yellow oil. This was flash-chromatographed on silica gel with toluene : ethyl acetate = 4:1 as eluent giving 3.6 g of E as colorless crystals with a m.p. of 53-58°C.

E: NMR (in $CDCl_3$): 3.9 ppm (s, 3H); 4.6 ppm (dq, 2H); 5.35 ppm (m, 1 H); 6.21 ppm (dd, 1 H); 6.9-7.05 ppm (m, 2H); 7.4-7.6 ppm (m, 2H); 7.78 ppm (dd, 1H).

*Compound F*

To a solution of 3.1 g of E (prepared above) in 100 ml of dichloromethane were added at O°C 17.8 ml of a 2.1 molar solution of $BCl_3$ in ethylene chloride. This mixture was stirred at O°C for 30 minutes and then poured on ice-water. The organic phase was washed 2× with water and 1× with sat. NaCl solution, dried with $Na_2S0_4$ and evaporated down. The residue was chromatographed on silica gel with toluene: ethyl acetate = 4:1 as eluent giving 2.1 g of F as a colorless oil.

F: NMR (in $CDCl_3$): 4.62 ppm (dq, 2H); 5.38 ppm (m, 1H); 6.27 ppm (dd, 1H); 6.8-7.0 ppm (m, 2H); 7.4-7.6 ppm (m, 2H); 7.76 ppm (dd, 1H); 10.42 ppm (s, 1H).

**EXAMPLE 3: Preparation of the Compound of Formula H and Derivatives**

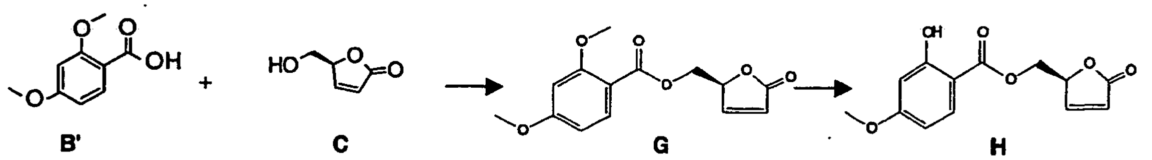

B' C G H

Compounds G and H were prepared using the above procedures.

G: NMR (in $CDCl_3$): 3.85 ppm (s, 3H); 3.88 ppm (s, 3H); 4.57 ppm (dq, 2H); 5.32 ppm (m, 1H); 6.19 ppm (dd, 1H); 6.4-6.55 ppm (m, 2H); 7.5 ppm (dd, 1H); 7.8 ppm (d, 1H).

H: NMR (in $CDCl_3$): 3.82 ppm (s, 3H); 4.39 ppm (m, 2H); 5.35 ppm (m, 1H); 6.23 ppm (dd, 1H); 6.35-6.5 ppm (m, 2H); 7.5 ppm (dd, 1H); 7.67 ppm (d, 1H); 10.6 ppm (s, 1H).

**EXAMPLE 4: Preparation of the Compound of Formula I and Derivatives**

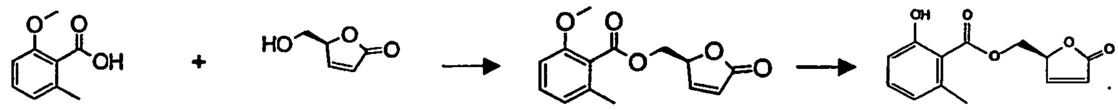

B C D I

Compound I was prepared using the above procedures.

I: NMR (in $CDCl_3$): 2.5 ppm (s, 3H); 4.5-4.8 ppm (m, 2H); 5.38 ppm (m, 1H); 6.27 ppm (dd, 1H); 6.7 ppm (d, 1H); 6.83 ppm (d, 1H); 7.29 ppm (t, 1H); 7.5 ppm (dd, 1H); 11.0 ppm (s, 1H).

The compounds of the invention have pharmacological properties. In particular, they show cytokine inhibitory effects, acting not only to inhibit the release of IL-1, IL-6 and TNF-$\alpha$, but also as functional antagonists of IL-1 as indicated in the following *in vitro* and *in vivo* test methods described below.

**EXAMPLE 5: Cytokine Release from THP-1 Cells**

The THP-1 cell line is generally available and is described by Tsuchiya et al. [Int. J. Cancer 26 171-176 (1980)]. 900 $\mu$l THP-1 cells (0.5 $\times$ $10^6$ cells) together with 100 U $\gamma$-interferon/0.9 ml RPMI 1640 medium (containing 2 mM L-glutamine and 5 % heat-inactivated fetal calf serum) are pipetted into 24-well culture plates. 100 $\mu$l of the compound to be tested are then added. After 3 hours at 37°C in 5 % $CO_2$/95 % air, 10 $\mu$l lipopolysaccharide (LPS) 500 $\mu$g/ml is added and the incubation continued for a further 40 hours. Appropriate controls (with and without stimulus, solvent) are also included. The media are then removed and clarified by centrifugation at 1000 g for 10 min. 1.0 ml digitonin 0.01 % is added to the wells to lyse the cells which are loosened by scraping with a rubber policeman and left at 4°C for 10 min. Lactate dehydrogenase measurements are then performed immediately and the samples stored at -20°C until the other determinations can be made. The assays are: IL-1$\beta$ (medium and lysate), IL-6 (medium), TNF-$\alpha$ (medium), $PGE_2$ (medium and lysate), lactate dehydrogenase (LDH) (medium and lysate) and DNA (lysates). IL-1$\beta$, IL-6 and TNF-$\alpha$ assays are determined using commercially available ELISA kits (Cistron), $PGE_2$ is measured using a standard RIA and

DNA fluorimetrically using DAPI (4', 6-diamidino-2-phenylindole·2HCl). In this test, the compounds of the invention inhibit IL-1β, IL-6, TNF-α and $PGE_2$ release at a concentration of about 0.1 to 10 μM. In contrast DNA levels remain substantially unaffected, and the compounds are non-toxic, since LDH release is unchanged.

**EXAMPLE 6: Cytokine Release from Human Monocytes**

*Human Monocytes*

Mononuclear cells are obtained from the blood of healthy volunteers via centrifugation and cultivated on tissue culture dishes with the test compound at various concentrations [Schnyder et al., Agents & Actions, **30**, 350-362 (1990)]. The non-adherent lymphocytes are removed after 4 hrs by washing several times. Fresh medium, test compound and LPS (10 μg/ml) as stimulant are added and the monocytes incubated for a.further day. The pooled culture media are diluted 1:10 with fresh medium and added to confluent rabbit chondrocytes. Metalloproteinase activity in the chondrocyte culture medium is assayed after a further 2 days as described below. Compounds of the invention are active in suppressing monokine release in this test method at a concentration of the order of from 0.1 to 10 μM.

*Determination of IL-1 by the chondrocyte test*

Purified IL-1, recombinant human IL-1-β (rhIL-1) or conditioned media collected from stimulated human monocytes, mouse macrophages or mouse cell line $P388D_1$, cause characteristic changes in the secretory pattern of chondrocytes. In particular, a latent metalloproteinase is induced, whilst secretion of plasminogen activator is reduced. The property of the metalloproteinase or stromelysin has been described in detail [Chin et al., J. Biol. Chem. **260,** 12367-12376 (1985)], as has that of the plasminogen activator [Schnyder et al., Anal. Biochem. **200,** 156-162 (1992)]. Dose-response curves using purified or recombinant IL-1 and neutralisation with an antibody to human monocyte IL-1 have shown that this system can be used as a specific and sensitive bioassay for IL-1. Stimulation of the secretion of a latent metalloproteinase by rabbit articular chondrocytes is relatively IL-1-specific, IL-2, TNF-α, recombinant human interferons-α, and -γ, phorbol myristate acetate, Concanavalin A, E-type prostaglandin and indomethacin having no influence [Schnyder and Payne, Brit. J. Rheumatol. **24** (suppl. 1), 128-132 (1985); Schnyder et al., J. Immunol. **138**, 496-503 (1987)].

Chondrocytes are harvested and cultured as described [Evequoz et al., Biochem. J. **219**, 667-677 (1984)]. Briefly, chondrocytes are released from slices of distal femur articular cartilage from ca. 1.2 kg female New Zealand White rabbits by treatment with proteinases. The washed cells are cultured on 48-well culture plates in DMEM, enriched with 1 % antibiotics, 2 mM glutamine and 10 % heat-inactivated fetal calf serum. After reaching confluency the cells are incubated with 20 μl samples of the test culture media for IL-1 bioassay and made up to a volume of 300 μl Iscove's modified Dulbecco's medium. The supernatant media are collected after 48 h, centrifuged and processed for biochemical analysis.

*Biochemical Assays*

Metalloproteinase is measured kinetically by using a 96-well plate Twinreader (Flow Laboratories AG) linked to a personal computer.

Ac-Pro-Leu-Gly-S-Leu-Leu-Gly-$OC_2H_5$, a synthetic substrate for vertebrate collagenase is used for the determination of metalloproteinase [Weingarten and Feder, Anal.

Biochem. 147, 437-440 (1985)]. 50 μl of the latent metalloproteinase is activated with 50 μl trypsin (120 μg/ml in 50 mM PIPES pH 6.8, containing 20 mM $CaCl_2$) for 30 min at 37°C, after which time the activities of all serine proteinases are stopped by adding 150 μl Soybean trypsin inhibitor (SBTI; 166 μg/ml in the above buffer). A 50 μl aliquot of the activated metalloproteinase is then mixed with 100 μl reagent solution (2.5 mM 5, 5'-dithio-bis-2-nitrobenzoic acid, 100 μg/ml SBTI, 20 mM $CaCl_2$ in the above buffer), and kept for 10 min at room temperature in order to react with all free SH-groups. The reaction is then started by adding 100 μl substrate solution (1.25 mM in buffer containing 100 μg/ml SBTI) and the changes in absorbance at 414 nm is measured 11 times at 1 min intervals.

**EXAMPLE 7: Functional IL-1 Antagonistic Effects**

The test method described in Example 6 above is repeated, but instead of adding human monocytes to the chondrocyte culture, IL-1 itself (recombinant human IL-1, Sandoz) is added at a concentration of 1 ng/ml. Metalloproteinase activity in the chondrocyte culture medium is assayed after 2 days. Compounds of the invention are active as functional IL-1 antagonists at concentrations of 1-10 μM.

**EXAMPLE 8: LPS-Fever**

An LPS-suspension (Sigma, No. L-5886; 100 μg/5 ml glucose solution/kg s.c.) is injected in male Tuttlingen SD rats (150-160 g). Two hours later the body temperature is measured using a thermistor rectal probe connected to an ELLAB telethermometer. After 4 hrs the test compound is administered. Two hours later (6 hrs after LPS administration) the temperature is measured again. The temperature increment shown by the untreated controls is taken as 100 % and that in the treated group is expressed as a percentage of this value. The $ED_{50}$ is the dose causing a 50 % inhibition of the temperature increase determined in the control rats. Compounds of the invention typically have an $ED_{50}$ in this assay of from about 0.1 to about 1 mg/kg.

**EXAMPLE 9: Carrageenan-Induced Paw Edema in the Rat**

Five OFA male rats, 150-170 g body weight, are used for each group. The test compound is administered orally as a suspension in physiological saline/0.5 % tragacanth one hour prior to the carrageenan injection. Carrageenan (0.1 ml of a 1 % suspension in physiological saline) is given by subplantar injection into one hind paw. The swelling of the paw is measured by means of an antiphlogometer according to Kemper & Amelm. A control reading is taken immediately after the injection, and the swelling is measured after 3 and 5 hrs. The mean value of the 3- and 5-hour reading is taken after deduction of the control reading, and the values obtained from the treated animals are expressed as a percentage of the value obtained from non-treated controls. The $ED_{50}$ is the dose causing a 50 % inhibition of the carrageenan-induced swelling after 3 hr. Compounds of the invention typically have $ED_{50}$s in this assay of from about 0.5 to about 1 mg/kg.

**EXAMPLE 10: Reporter Gene Assay for Compounds which Destabilize mRNA**

The THP-1 cell lines, clone No. 63 (containing PGL2_Neo30) and clone No. 53 (containing pGL2-Control) are grown, differentiated and stimulated with IFN-γ and LPS identical to normal THP-1 cells (for details see WO 00/39314). Compound is added 16 hrs after the addition of LPS and cell extracts are then taken 8 hrs later or as indicated. Luciferase activity was inhibited by 1 μM benzoic acid derivative on average by 40 %, whereas up to 5 μM of benzoic acid derivative had no effects on the control clone No. 53.

**Table 1**

| *COMPOUND* | *Luciferase reporter gene assay (% control)* | | | |
| --- | --- | --- | --- | --- |
| | clone | 0.5 μM | 1 μM | 5 μM |
| 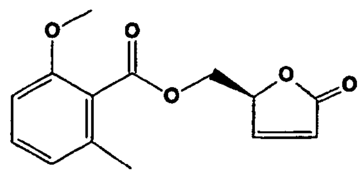 | 63 | 85 | 79 | 29 |
| | 53 | 89 | 93 | 99 |

The compounds of the invention are therefore indicated for the treatment of disorders with an etiology associated with or comprising excessive cytokine release, particularly IL-1 release, e.g. in a wide variety of inflammatory states and diseases such as rheumatoid arthritis, osteoarthritis, septic shock, psoriasis, atherosclerosis, inflammatory bowel disease, Crohn's disease and asthma.

For the above uses the required dosage will of course vary depending on the mode of administration, the particular condition to be treated and the effect desired. In general however, satisfactory results are achieved at dosage rates of from about 0.1 to 20 mg/kg animal body weight, preferably 0.5 to 5 mg/kg.

**EXAMPLE 11: Preparation of a Pharmaceutical Composition**

Tablets, each comprising e.g. 50 mg of compound or a pharmaceutically acceptable salt, are prepared as follows:

| Composition (10000 tablets) | |
| --- | --- |
| active ingredient | 500.0 g |
| lactose | 500.0 g |

(continued)

| | |
|---|---|
| potato starch | 352.0 g |
| gelatin | 8.0 g |
| talc | 60.0 g |
| magnesium stearate | 10.0 g |
| silicon dioxide (highly dispersed) | 20.0 g |
| ethanol | q.s. |

The active ingredient is mixed with the lactose and 292 g of potato starch and the mixture is moistened with an ethanolic solution of the gelatin and granulated through a sieve. After drying, the remainder of the potato starch, the magnesium stearate, the talc and the silicon dioxide are mixed in and the mixture is compressed to form tablets, each weighing 145.0 mg and comprising 50.0 mg of active ingredient; the tablets may, if desired, be provided with breaking notches for finer adaptation of the dose.

**EXAMPLE 12: Preparation of a Pharmaceutical Composition**

Film-coated tablet, each comprising 100 mg of benzoic acid derivatives or a pharmaceutically acceptable salt are prepared as follows:

| Composition (for 1000 film-coated tablets) | |
|---|---|
| active ingredient | 100.0 g |
| lactose | 100.0 g |
| corn starch | 70.0 g |
| talc | 60.0 g |
| calcium stearate | 1.5 g |
| hydroxypropylmethylcellulose | 2.36 g |
| shellac | 0.64 g |
| water | q.s |
| methylene chloride | q.s. |

The active ingredient, the lactose and 40 g of the corn starch are mixed and moistened with a paste prepared from 15 g of corn starch and water (with heating) and granulated. The granules are dried, the remainder of the corn starch, the talcum and the calcium stearate are added and mixed with the granules. The mixture is compressed to form tablets (weight: 280 mg) which are then film-coated with a solution of the hydroxypropylmethylcellulose and the shellac in methylene chloride; final weight of the film-coated tablet: 283 mg.

**EXAMPLE 13: Preparation of a Pharmaceutical Composition**

Hard gelatin capsules, comprising 100 mg of active ingredient, for example benzoic acid derivatives or a pharmaceutically acceptable salt are prepared, for example, as follows:

| Composition (for 1000 capsules) | |
|---|---|
| active ingredient | 100.0 g |
| lactose | 250.0 g |
| microcrystalline cellulose | 30.0 g |
| sodium lauryl sulfate | 2.0 g |
| magnesium stearate | 8.0 g |

The sodium lauryl sulfate is added to the lyophilized active ingredient through a sieve of 0.2 mm mesh size. The two components are intimately mixed. Then first the lactose is added through a sieve of 0.6 mm mesh size and then the microcrystalline cellulose is added through a sieve of 0.9 mm mesh size. The mixture is then intimately mixed again for 10 minutes. Finally the magnesium stearate is added through a sieve of 0.8 mm mesh size. After mixing for a further 3 minutes, size 0 hard gelatin capsules are each filled with 390 mg of the resulting formulation. Soft gelatin capsules may be prepared using similar ingredients and procedures.

**EXAMPLE 14: Effect on Cell Viability of Cancer Cells**

The cell lines used in this experiment (MCF-7, MDA-MB-231, HCT116, KB 31, HCT-15, and DU 145 were obtained from ATCC, Bethesda, MD, U.S.A. Exponentially growing cells were treated with various concentrations of the test compound. The cells were further incubated for 24 h, 48h and 96 h. Cells were then stained with YOPRO-1 as described in T. Idziorek et al., J Immunol Meth 185, 249 (1995 and the percentage of dying or dead cells was determined.

**EXAMPLE 15: Effect on Cell Cycle Progression of Cancer Cells**

The cell lines used in this experiment (MCF-7, MDA-MB-231, HCT116, KB 31, HCT-15, and DU 145 were obtained from ATCC, Bethesda, MD, U.S.A. Exponentially growing cells were treated with various concentrations of the test compound. The cells were further incubated for 24 h, 48h and 96 h. Cells were then lysed and dsDNA was stained with YOPRO-1 as described in T. Idziorek et al., J Immunol Meth 185, 249 (1995). Fluorecsence measurements were carried out essentially as described in V.L. Singer et al., Anal Biochem 249, 228 (1997) and values compared to control cells.

**EXAMPLE 16: Effect on interleukin-1β mRNA levels**

Total cellular RNA was isolated from THP-1 cells at different times after the initiation of differentiation using the guanidine isothiocyanate method of Chirgwin *et al.* (44). 20 μg of each RNA sample was denatured and analyzed on a 1% agarose gel containing 2.2 M formaldehyde. The gel was transferred to Hybond-N (Amersham), UV-crosslinked by the Stratagene Stratalinker (1600 μJoules × 100) and judged for equal loading of RNA by methylene blue staining. Blots were hybridized to a digoxigenin (DIG)-labeled DNA probe. Labeling and detection was carried out by the DIG kit (Boehringer Mannheim) according to the manufacturer's specifications. DNA probes were obtained by subcloning RT-PCR derived fragments of the genes of interest, into pGemT-vector (Promega). For the RT-PCR analysis, 1 μg of total RNA was reverse transcribed using AMV reverse transcriptase (Life Sciences Inc., St. Petersburg, Florida), then amplified by the PCR method. Each PCR reaction also contained a set of β-actin primers as internal control. The levels of the expected PCR product were compared to the constant levels of β-actin. The size of the PCR products was compared to MspI digested pBR322 standards (New England Biolabs). PCR products were analyzed on a 4% agarose gel. Nucleotide sequences for the oligonucleotide 5' and 3' primers, respectively, were:

| | 5' | 3' |
|---|---|---|
| β-actin: | ATGGGTCAGAAGGATTCCTA | AGAGGCGTACAGGGATAGCAC |
| IL-1β: | GACACATGGGATAACGAGGCT | ACGCAGGACAGGTACAGATTC |

Compounds of the invention are reducing mRNA levels of interleukin-1β up to 70% at concentrations of 10 μM.

## Claims

1. A compound having structural formula (I):

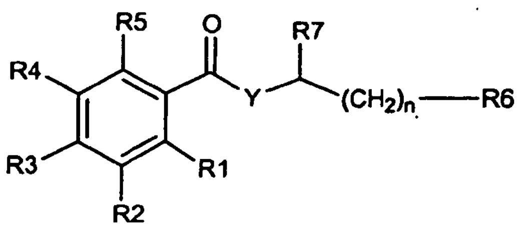

(I)

or stereoisomers thereof, or pharmaceutically acceptable salts or hydrates thereof or physiologically acceptable and hydrolysable esters thereof, wherein:

R1 is H, OH, halogen, $(C_1\text{-}C_4)$ alkyl, up to $C_4$ alkenyl, $(C_1\text{-}C_4)$ alkoxy, or $(C_1\text{-}C_4)$ alkyl-COO-;
R2 is H, OH, halogen, $(C_1\text{-}C_4)$ alkyl, up to $C_4$ alkenyl, $(C_1\text{-}C_4)$ alkoxy, or $(C_1\text{-}C_4)$ alkyl-COO-;
R3 is H, OH, halogen, $(C_1\text{-}C_4)$ alkyl, up to $C_4$ alkenyl, $(C_1\text{-}C_4)$ alkoxy, or $(C_1\text{-}C_4)$ alkyl-COO-;
R4 is H, OH, halogen, $(C_1\text{-}C_4)$ alkyl, up to $C_4$ alkenyl, $(C_1\text{-}C_4)$ alkoxy, or $(C_1\text{-}C_4)$ alkyl-COO-;
R5 is H, OH, halogen, $(C_1\text{-}C_4)$ alkyl, up to $C_4$ alkenyl, $(C_1\text{-}C_4)$ alkoxy, or $(C_1\text{-}C_4)$ alkyl-COO-;
Y is O or -NR', wherein R' is H or $(C_1\text{-}C_4)$ alkyl;
n is 0-8;
R6 is a 5 to 8 membered lactone or lactam ring;
R7 is H, $(C_1\text{-}C_4)$ alkyl, up to $C_4$ alkenyl, $(C_1\text{-}C_4)$ alkoxy, phenyl or $(C_1\text{-}C_4)$ alkyl-COO-,

wherein the lactam or lactone ring optionally comprises one or more C=C double bonds and the lactam or lactone ring is optionally substituted with one or more $(C_1\text{-}C_4)$alkyl or up to $C_4$ alkenyl groups;
with the proviso that when R1 = R2 = R3 = R4 = R5 = R7 = H, and Y is O, and n is 0, then R6 is other than

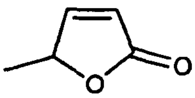 , 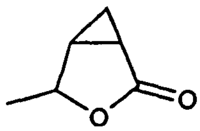 , 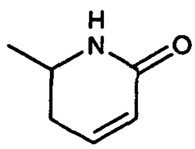 or 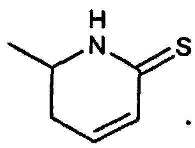 .

**2.** The compound according to claim 1, wherein:

R1 = H, OH, MeCOO- or Methoxy;
R2 = H;
R3 = H, OH, MeCOO- or Methoxy;
R4 = H, Methoxy or halogen;
R5 = H, OH, Methoxy or $(C_1\text{-}C_4)$ alkyl;
Y is O;
R6 is

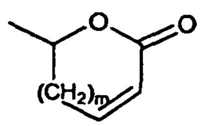

wherein, m is 0-4 ;
R7 = H, Methyl, Isopropyl.

**3.** The compound according to claim 1, wherein:

R1 is H, -OH, MeO- or MeCOO-;
R2 is H;
R3 is H, -OH, MeO- or MeCOO-;
R4 is H or halogen;
R5 is -OH, MeO-, MeCOO-or Methyl.

**4.** The compound according to claim 2, wherein:

R1 is H or MeO, $R_3$ is H, -OH or MeO-; $R_4$ is H and $R_5$ is Methoxy or Methyl.

**5.** The compound according to claim 1, wherein said compound is selected from the group of compounds:

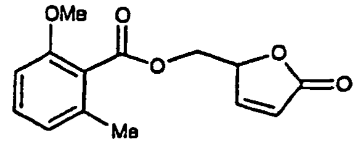
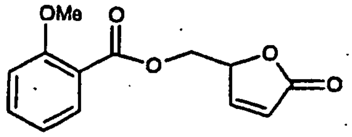
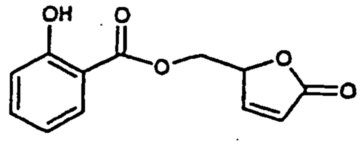
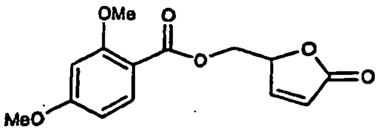
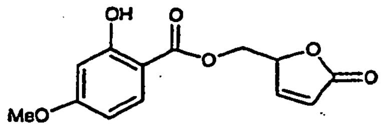
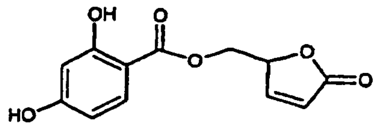
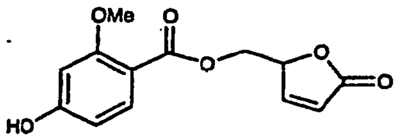
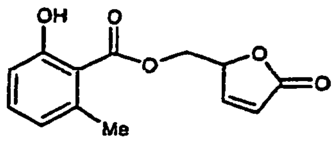

**6.** The compound according to claim 1, wherein said compound is selected from the group of compounds:

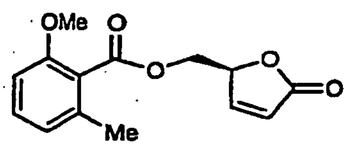
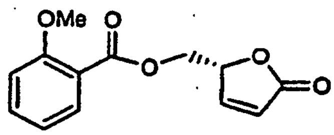
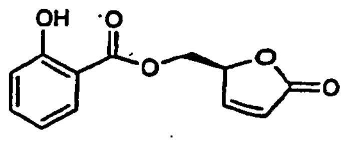
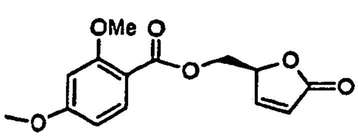
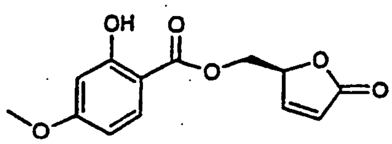
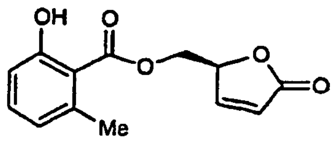

**7.** A process for the preparation of a compound of Formula I, according to claim 1, a pharmaceutically acceptable metabolically labile ester or amide thereof, or a pharmaceutically acceptable salt thereof, which comprises:

a) reacting a compound of formula (II):

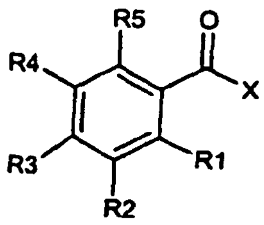

(II)

wherein:

R1 is H, OH, halogen, ($C_1$-$C_4$) alkyl, up to $C_4$ alkenyl, ($C_1$-$C_4$) alkoxy, or ($C_1$-$C_4$)alkyl-COO-;
R2 is H, OH, halogen, ($C_1$-$C_4$) alkyl, up to $C_4$ alkenyl, ($C_1$-$C_4$) alkoxy, or ($C_1$-$C_4$) alkyl-COO-;
R3 is H, OH, halogen, ($C_1$-$C_4$) alkyl, up to $C_4$ alkenyl, ($C_1$-$C_4$) alkoxy, or ($C_1$-$C_4$) alkyl-COO-;
R4 is H, OH, halogen, ($C_1$-$C_4$) alkyl, up to $C_4$ alkenyl, ($C_1$-$C_4$) alkoxy, or ($C_1$-$C_4$) alkyl-COO-; and
X is halogen;

with a compound of formula (III):

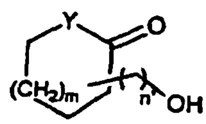

(III)

wherein:

n' is 1-9; and
m is 0-4;
compound (III) optionally contains one or more C=C double bonds and is substituted with one or more ($C_1$-$C_4$) alkyl or ($C_1$-$C_4$) alkenyl group; or

b) reacting a compound of formula (IV):

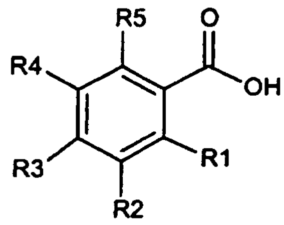

(IV)

wherein R1-R5 are as defined above;
with a compound of formula (II) in the presence of an acid under Azeotropic distillation conditions; or
c) reacting an alkali or alkaline earth metal salt of compound of formula (IV):

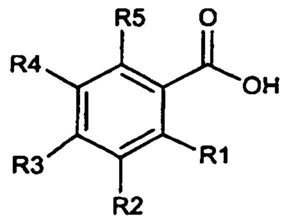

(IV)

wherein R1-R5 are as defined above;
with a compound of formula (V):

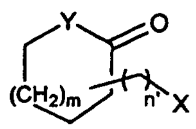

(V)

wherein;

X is F, Cl, Br or I;
n' is 1-9; and
m is 0-4; and compound of formula (V) may optionally contain one or more double bonds and is optionally substituted with one or more ($C_{1-}C_4$) alkyl or up to $C_4$ alkenyl group.

**8.** A pharmaceutical composition comprising a compound having structural formula (I):

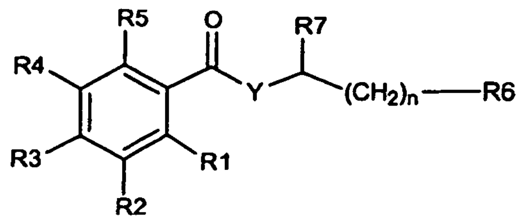

(I)

a stereoisomer thereof, or a pharmaceutically acceptable salt or hydrate thereof or a physiologically acceptable and a hydrolysable ester thereof and a pharmaceutically acceptable carrier, diluent or excipient;
wherein:

R1 is H, OH, halogen, ($C_1$-$C_4$) alkyl, up to $C_4$ alkenyl, ($C_1$-$C_4$) alkoxy, or ($C_1$-$C_4$) alkyl-COO-;
R2 is H, OH, halogen, ($C_1$-$C_4$) alkyl, up to $C_4$ alkenyl, ($C_1$-$C_4$) alkoxy, or ($C_1$-$C_4$) alkyl-COO-;
R3 is H, OH, halogen, ($C_1$-$C_4$) alkyl, up to $C_4$ alkenyl, ($C_1$-$C_4$) alkoxy, or ($C_1$-$C_4$) alkyl-COO-;
R4 is H, OH, halogen, ($C_1$-$C_4$) alkyl, up to $C_4$ alkenyl, ($C_1$-$C_4$) alkoxy, or ($C_1$-$C_4$) alkyl-COO-;
R5 is H, OH, halogen, ($C_1$-$C_4$) alkyl, up to $C_4$ alkenyl, ($C_1$-$C_4$) alkoxy, or ($C_1$-$C_4$) alkyl-COO-;
Y is O or -NR', wherein R' is H or ($C_1$-$C_4$) alkyl;
n is 0-8;
R6 is a 5 to 8 membered lactone or lactam ring;
R7 is H, ($C_1$-$C_4$) alkyl, up to $C_4$ alkenyl, ($C_1$-$C_4$) alkoxy, phenyl or ($C_1$-$C_4$) alkyl-COO-;

wherein the lactam or lactone ring optionally comprises one or more C=C double bonds and the lactam or lactone ring is optionally substituted with one or more ($C_1$-$C_4$) alkyl or up to $C_4$ alkenyl groups; for the prophylaxis or treatment of a cancer and/or malignant disease, or an inflammatory disease or medical condition; or for the prophylaxis or treatment of a disease or medical condition selected from the group comprising: acute and/or chronic inflammation, autoimmune diseases, respiratory diseases, infectious diseases, transplant rejection, neuronal inflammation, Alzheimer's disease and cardiovascular diseases; or for the prophylaxis or treatment of oncogene mediated cancers or malignant diseases; or for the prophylaxis or treatment of tumour growth and/or metastasis invasion in general; or for the prevention or reversal of multi-drug resistance.

**9.** A pharmaceutical composition comprising one or more compounds according to any one of claims 1-6 and a pharmaceutically acceptable carrier, diluent or excipient.

**10.** The composition according to claim 8 or 9 additionally comprising a cytostatic or a cytotoxic agent.

**11.** The composition according to claim 8 or 9 additionally comprising a chemotherapeutic agent.

**12.** Use of a compound having structural formula (I):

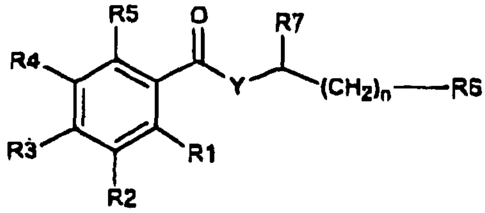

(I)

or stereoisomers thereof, or pharmaceutically acceptable salts or hydrates thereof or physiologically acceptable and hydrolysable esters thereof, in the manufacture of a medicament for the treatment of a mammal,
wherein:

R1 is H, OH, halogen, ($C_1$-$C_4$) alkyl, up to $C_4$ alkenyl, ($C_1$-$C_4$) alkoxy, or ($C_{1-}C_4$) alkyl-COO-;
R2 is H, OH, halogen, ($C_1$-$C_4$) alkyl, up to $C_4$ alkenyl, ($C_1$-$C_4$) alkoxy, or ($C_{1-}C_4$) alkyl-COO-;
R3 is H, OH, halogen, ($C_1$-$C_4$) alkyl, up to $C_4$ alkenyl, ($C_1$-$C_4$) alkoxy, or ($C_{1-}C_4$) alkyl-COO-;
R4 is H, OH, halogen, ($C_1$-$C_4$) alkyl, up to $C_4$ alkenyl, ($C_1$-$C_4$) alkoxy, or ($C_{1-}C_4$) alkyl-COO-;
R5 is H, OH, halogen, ($C_1$-$C_4$) alkyl, up to $C_4$ alkenyl, ($C_1$-$C_4$) alkoxy, or ($C_{1-}C_4$) alkyl-COO-;
Y is O or -NR', wherein R' is H or ($C_1$-$C_4$) alkyl;
n is 0-8;
R6 is a 5 to 8 membered lactone or lactam ring;
R7 is H, ($C_1$-$C_4$) alkyl, up to $C_4$ alkenyl, ($C_1$-$C_4$) alkoxy, phenyl or ($C_1$-$C_4$) alkyl-COO-,

wherein the lactam or lactose ring optionally comprises one or more C=C double bonds and the lactam or lactone ring is optionally substituted with one or more ($C_1$-$C_4$)alkyl or up to $C_4$ alkenyl groups.

**13.** Use of the compound according to any one of claims 1 - 6 in the manufacture of a medicament for the treatment of a mammal.

**14.** The use according to claim 12 or 13, wherein said medicament is used in the treatment of a cancer and/or a malignant disease.

**15.** The use according to claim 14, wherein said cancer and/or malignant disease comprises an abnormal cell proliferation of malignant cells of various tissues and/or organs selected from the group comprising: muscle, bone or connective tissues, the skin, brain, lungs, sex organs, the lymphatic or renal systems, mammary or blood cells, liver, the digestive tract, pancreas and thyroid or adrenal glands.

**16.** The use according to claim 14, wherein said cancer and/or malignant disease is selected from the group comprising: solid tumours, cancers of the ovary, breast, brain, prostate, colon, stomach, kidney or testicles, Kaposi's sarcoma, cholangioma, chorioma, neuroblastoma, Wilms' tumour, Hodgkin's disease, melanomas, multiple myelomas, lymphatic leukemias and acute and/or chronic granulocytic lymphomas.

**17.** The use according to claim 12 or 13, wherein said medicament is used in the treatment of an inflammatory disease or medical condition.

**18.** The use according to claim 17 wherein said disease or medical condition is selected from the group comprising: rheumatoid arthritis, osteoartbritis, septic shock, psoriasis, atherosclerosis, inflammatory bowel disease, Crohn's disease and asthma.

**19.** The use according to claim 12 or 13, wherein the medicament is used in the treatment of a disease or medical condition selected from the group comprising: acute and/or chronic inflammation, autoimmune diseases, respiratory diseases, infectious diseases, transplant rejection, neuronal inflammation, Alzheimer's disease and cardiovascular diseases.

**20.** The use according to claim 12 or 13, wherein the medicament is used in the treatment and prevention of oncogene mediated cancers and malignant diseases, to treat or prevent tumour growth and/or metastasis invasion in general, or to prevent or reverse multidrug resistance.

**21.** The use according to any one of claims 12 - 20, wherein said mammal is a human.

**22.** A compound having structural formula (I):

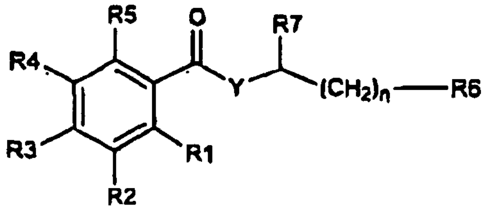

(I)

or stereoisomers thereof, or pharmaceutically acceptable salts or hydrates thereof or physiologically acceptable and hydrolysable esters thereof, wherein:

R1 is H, OH, halogen, ($C_1$-$C_4$) alkyl, up to $C_4$ alkenyl, ($C_1$-$C_4$) alkoxy, or ($C_1$-$C_4$) alkyl-COO-;
R2 is H, OH, halogen, ($C_1$-$C_4$) alkyl, up to $C_4$ alkenyl, ($C_1$-$C_4$) alkoxy, or ($C_1$-$C_4$) alkyl-COO-;
R3 is H, OH, halogen, ($C_1$-$C_4$) alkyl, up to $C_4$ alkenyl, ($C_1$-$C_4$) alkoxy, or ($C_1$-$C_4$) alkyl-COO-;
R4 is H, OH, halogen, ($C_1$-$C_4$) alkyl, up to $C_4$ alkenyl, ($C_1$-$C_4$) alkoxy, or ($C_1$-$C_4$) alkyl-COO-;
R5 is H, OH, halogen, ($C_1$-$C_4$) alkyl, up to $C_4$ alkenyl, ($C_1$-$C_4$) alkoxy, or ($C_1$-$C_4$) alkyl-COO-;
Y is O or -NR', wherein R' is H or ($C_1$-$C_4$) alkyl;
n is 0-8;
R6 is a 5 to 8 membered lactone or lactam ring;
R7 is H, ($C_1$-$C_4$) alkyl, up to $C_4$ alkenyl, ($C_1$-$C_4$) alkoxy, phenyl or ($C_1$-$C_4$) alkyl-COO-,

wherein the lactam or lactone ring optionally comprises one or more C=C double bonds and the lactam or lactone ring is optionally substituted with one or more ($C_1$-$C_4$)alkyl or up to $C_4$ alkenyl groups,
for the prophylaxis or treatment of a cancer and/or malignant disease.

**23.** The compound according to any one of claims 1 - 6, for the prophylaxis or treatment of a cancer and/or malignant disease.

**24.** The compound according to claim 22 or 23, wherein said cancer and/or malignant disease comprises an abnormal cell proliferation of malignant cells of various tissues and/or organs selected from the group comprising: muscle, bone or connective tissues, the skin, brain, lungs, sex organs, the lymphatic or renal systems, mammary or blood cells, liver, the digestive tract, pancreas and thyroid or adrenal glands.

**25.** The compound according to claim 22 or 23, where said cancer and/or malignant disease is selected from the group comprising: solid tumours, cancers of the ovary, breast, brain, prostate, colon, stomach, kidney or testicles, Kaposi's sarcoma, cholangioma, chorioma, neuroblastoma, Wilms' tumour, Hodgkin's disease, melanomas, multiple myelomas, lymphatic leukemias and acute and/or chronic granulocytic lymphomas.

**26.** A compound having structural formula (I):

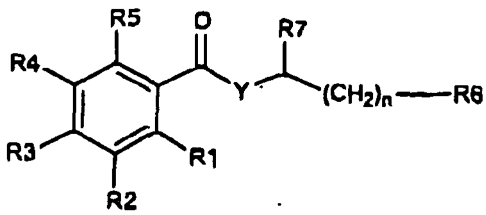

(I)

stereoisomers thereof, or pharmaceutically acceptable salts or hydrates thereof or physiologically acceptable and hydrolysable esters thereof, wherein:

R1 is H, OH, halogen, ($C_1$-$C_4$) alkyl, up to $C_4$ alkenyl, ($C_1$-$C_4$) alkoxy, or ($C_1$-$C_4$) alkyl-COO-;

R2 is H, OH, halogen, ($C_1$-$C_4$) alkyl, up to $C_4$ alkanyl, ($C_1$-$C_4$) alkoxy, or ($C_1$-$C_4$) alky-COO-;
R3 is H, OH, halogen, ($C_1$-$C_4$) alkyl, up to $C_4$ alkenyl, ($C_1$-$C_4$) alkoxy, or ($C_1$-$C_4$) alkyl-COO-;
R4 is H, OH, halogen, ($C_1$-$C_4$) alkyl, up to $C_4$ alkenyl, ($C_1$-$C_4$) alkoxy, or ($C_1$-$C_4$) alkyl-COO-;
R5 is H, OH, halogen, ($C_1$-$C_4$) alkyl, up to $C_4$ alkenyl, ($C_1$-$C_4$) alkoxy, or ($C_1$-$C_4$) alkyl-COO-;
Y is O or -NR', wherein R' is H or ($C_1$-$C_4$) alkyl;
n is 0-8;
R6 is a 5 to 8 membered lactone or lactam ring;
R7 is H, ($C_1$-$C_4$) alkyl, up to $C_4$ alkenyl, ($C_1$-$C_4$) alkoxy, phenyl or ($C_1$-$C_4$) alkyl-COO-,

wherein the lactam or lactone ring optionally comprises one or more C=C double bonds and the lactam or lactone ring is optionally substituted with one or more ($C_1$-$C_4$)alkyl or up to $C_4$ alkenyl groups,
for the prophylaxis or treatment of an inflammatory disease or medical condition.

**27.** The compound according to any one of claims 1 - 6, for the prophylaxis or treatment of an inflammatory disease or medical condition.

**28.** The compound according to claim 26 or 27, wherein said disease or medical condition is selected from the group comprising: rheumatoid arthritis, osteoarthritis, septic shock, psoriasis, atherosclerosis, inflammatory bowel disease, Crohn's disease and asthma.

**29.** A compound having structural formula (I):

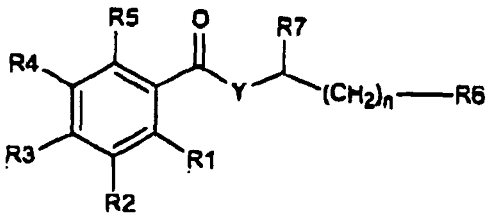

(I)

stereoisomers thereof, or pharmaceutically acceptable salts or hydrates thereof or physiologically acceptable and hydrolysable esters thereof, wherein:

R1 is H, OH, halogen, ($C_1$-$C_4$) alkyl, up to $C_4$ alkenyl, ($C_1$-$C_4$) alkoxy, or ($C_1$-$C_4$) alkyl-COO-;
R2 is H, OH, halogen, ($C_1$-$C_4$) alkyl, up to $C_4$ alkenyl, ($C_1$-$C_4$) alkoxy, or ($C_1$-$C_4$) alkyl-COO-;
R3 is H, OH, halogen, ($C_1$-$C_4$) alkyl, up to $C_4$ alkenyl, ($C_1$-$C_4$) alkoxy, or ($C_1$-$C_4$) alkyl-COO-;
R4 is H, OH, halogen, ($C_1$-$C_4$) alkyl, up to $C_4$ alkenyl, ($C_1$-$C_4$) alkoxy, or ($C_1$-$C_4$) alkyl-COO-;
R5 is H, OH, halogen, ($C_1$-$C_4$) alkyl, up to $C_4$ alkenyl, ($C_1$-$C_4$) alkoxy, or ($C_1$-$C_4$) alkyl-COO-;
Y is O or -NR', wherein R' is H or ($C_1$-$C_4$) alkyl;
n is 0-8;
R6 is a 5 to 8 membered lactone or lactam ring;
R7 is H, ($C_1$-$C_4$) alkyl, up to $C_4$ alkenyl, ($C_1$-$C_4$) alkoxy, phenyl or ($C_1$-$C_4$) alkyl-COO-,

wherein the lactam or lactone ring optionally comprises one or more C=C double bonds and the lactam or lactone ring is optionally substituted with one or more ($C_1$-$C_4$)alkyl or up to $C_4$ alkenyl groups, for the treatment and prevention of oncogene mediated cancers and malignant diseases, to treat or prevent tumour growth and/or metastasis invasion in general, or to prevent or reverse multidrug resistance.

**30.** The compound according to any one of claims 1 - 6, for the treatment and prevention of oncogene mediated cancers and malignant diseases, to treat or prevent tumour growth and/or metastasis invasion in general, or to prevent or reverse multidrug resistance.

**31.** A compound having structural formula (I):

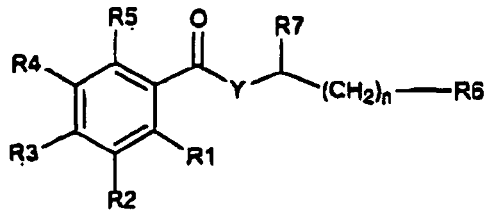

(I)

stereoisomers thereof, or pharmaceutically acceptable salts or hydrates thereof or physiologically acceptable and hydrolysable esters thereof, wherein:

R1 is H, OH, halogen, ($C_1$-$C_4$) alkyl, up to $C_4$ alkenyl, ($C_1$-$C_4$) alkoxy, or ($C_{1-}C_4$) alkyl-COO-;
R2 is H, OH, halogen, ($C_1$-$C_4$) alkyl, up to $C_4$ alkenyl, ($C_1$-$C_4$) alkoxy, or ($C_{1-}C_4$) alkyl-COO-;
R3 is H, OH, halogen, ($C_1$-$C_4$) alkyl, up to $C_4$ alkenyl, ($C_1$-$C_4$) alkoxy, or ($C_{1-}C_4$) alkyl-COO-;
R4 is H, OH, halogen, ($C_1$-$C_4$) alkyl, up to $C_4$ alkenyl, ($C_1$-$C_4$) alkoxy, or ($C_{1-}C_4$) alkyl-COO-;
R5 is H, OH, halogen, ($C_1$-$C_4$) alkyl, up to $C_4$ alkenyl, ($C_1$-$C_4$) alkoxy, or ($C_{1-}C_4$) alkyl-COO-;
Y is O or -NR', wherein R' is H or ($C_1$-$C_4$) alkyl;
n is 0-8;
R6 is a 5 to 8 membered lactone or lactam ring;
R7 is H, ($C_1$-$C_4$) alkyl, up to $C_4$ alkenyl, ($C_1$-$C_4$) alkoxy, phenyl or ($C_1$-$C_4$) alkyl-COO-,

wherein the lactam or lactone ring optionally comprises one or more C=C double bonds and the lactam or lactone ring is optionally substituted with one or more ($C_1$-$C_4$)alkyl or up to $C_4$ alkenyl groups, for treating or preventing a disease or medical condition selected from the group comprising: acute and/or chronic inflammation, autoimmune diseases, respiratory diseases, infectious diseases, transplant rejection, neuronal inflammation, Alzheimer's disease and cardiovascular diseases.

**32.** The compound according to any one of claims 1 - 6, for treating or preventing a disease or medical condition selected from the group comprising: acute and/or chronic inflammation, autoimruune diseases, respiratory diseases, infectious diseases, transplant rejection, neuronal inflammation, Alzheimer's disease and cardiovascular diseases.

**33.** The compound according to any one of claims 22 - 32, for treatment of a human.

**Patentansprüche**

**1.** Verbindung mit der Strukturformel (I):

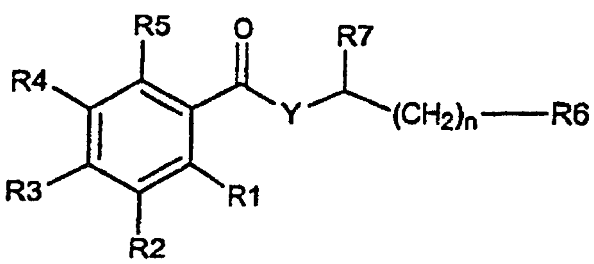

(I)

oder Stereosisomere davon, oder pharmazeutisch verträgliche Salze oder Hydrate davon oder physiologisch verträgliche und hydrolysierbare Ester davon, worin:

R1 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R2 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;

R3 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R4 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R5 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
Y für O oder -NR' steht, worin R' für H oder $(C_1-C_4)$-Alkyl steht;
n für 0 - 8 steht;
R6 für einen 5- bis 8- gliedrigen Lacton- oder Lactamring steht;
R7 für H, $(C_1-C_4)$-Alkyl, bis zu $C_4$-Alkenyl, $(C_1-C_4)$-Alkoxy, Phenyl oder $(C_1-C_4)$-Alkyl-COO- steht;

worin der Lactam- oder Lactonring optional eine oder mehr C=C-Doppelbindung(en) umfasst und der Lactam- oder Lactonring optional mit einer oder mehr $(C_1-C_4)$-Alkyl- oder bis zu $C_4$-Alkenylgruppe(n) subsituiert ist; unter der Voraussetzung, dass wenn R1 = R2 = R3 = R4 = R5 = R7 = H darstellt, und Y für O steht, und n für 0 steht, R6 dann anders als

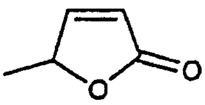 , 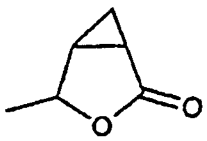 , 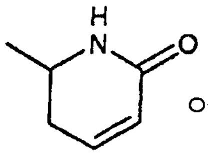 oder 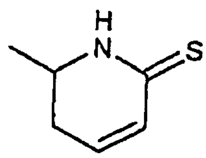

ist.

2. Verbindung nach Anspruch 1, worin:

R1 = H, OH, MeCOO- oder Methoxy darstellt;
R2 = H darstellt;
R3 = H, OH, MeCOO- oder Methoxy darstellt;
R4 = H, Methoxy oder Halogen darstellt;
R5 = H, OH, Methoxy oder $(C_1-C_4)$-Alkyl darstellt;
Y für O steht;
R6

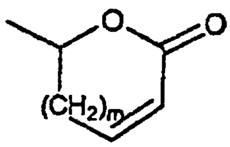

darstellt, worin m für 0 - 4 steht;
R7 = H, Methyl, Isopropyl darstellt.

3. Verbindung nach Anspruch 1, worin:

R1 für H, -OH, MeO- oder MeCOO- steht;
R2 für H steht;
R3 für H, -OH, MeO- oder MeCOO- steht;
R4 für H oder Halogen steht;
R5 für -OH, MeO-, MeCOO- oder Methyl steht.

4. Verbindung nach Anspruch 2, worin:

R1 für H oder MeO- steht, $R_3$ für H, -OH oder MeO- steht; $R_4$ für H steht und $R_5$ für Methoxy oder Methyl steht.

5. Verbindung nach Anspruch 1, worin die genannte Verbindung aus der folgenden Gruppe von Verbindungen ausgewählt ist:

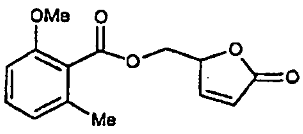 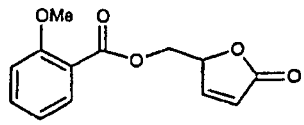 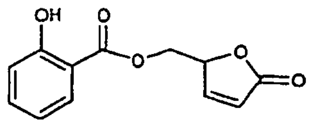

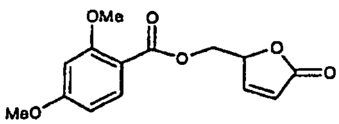 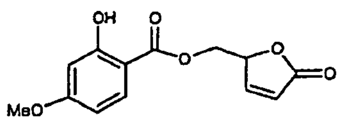 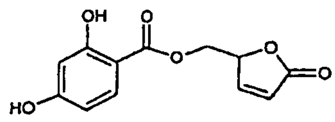

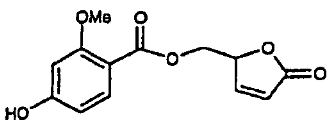 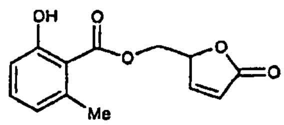

**6.** Verbindung nach Anspruch 1, worin die genannte Verbindung aus der folgenden Gruppe von Verbindungen ausgewählt ist:

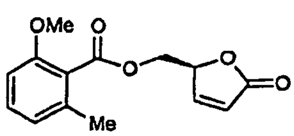 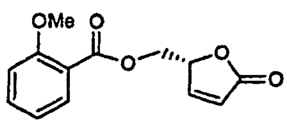 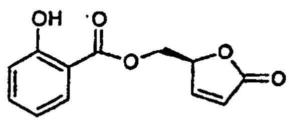

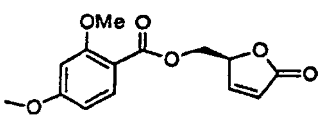 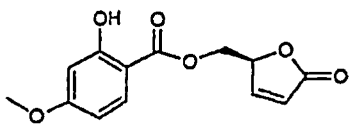 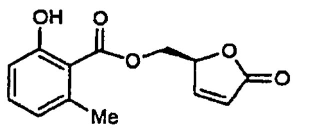

**7.** Verfahren zur Herstellung einer Verbindung der Formel I nach Anspruch 1, eines pharmazeutisch verträglichen, metabolisch labilen Esters oder eines Amids davon, oder eines pharmazeutisch verträglichen Salzes davon, das Folgendes umfasst:

a) Zur Reaktion bringen einer Verbindung der Formel (II):

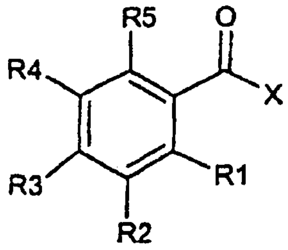

(II)

worin:

R1 für H, OH, Halogen, ($C_1$-$C_4$)-Alkyl, bis zu $C_4$-Alkenyl, ($C_1$-$C_4$)-Alkoxy oder ($C_1$-$C_4$) -Alkyl-COO- steht;
R2 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R3 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R4 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht; und
X für Halogen steht;

mit einer Verbindung der Formel (III):

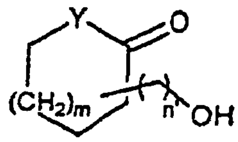

(III)

worin:

n' für 1 - 9 steht; und
m für 0 - 4 steht;
Verbindung (III) optional eine oder mehr C=C-Doppelbindung(en) enthält und mit einer oder mehr ($C_1$-$C_4$)-Alkyl- oder ($C_1$-$C_4$)-Alkenylgruppe(n) substituiert ist; oder

b) zur Reaktion bringen einer Verbindung der Formel (IV):

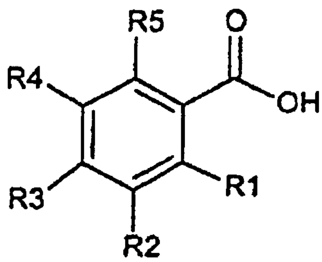

(IV)

worin R1-R5 wie vorstehend definiert sind;

mit einer Verbindung der Formel (II) in Anwesenheit einer Säure unter azeotropen Destillationsbedingungen; oder

c) zur Reaktion bringen eines Alkali- oder Erdalkalimetallsalzes der Verbindung der Formel (IV):

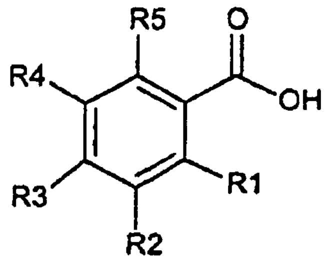

(IV)

worin R1-R5 wie vorstehend definiert sind;
mit einer Verbindung der Formel (V):

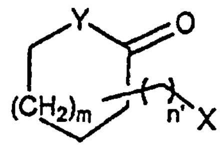

(V)

worin:

X für F, Cl, Br oder I steht;
n' für 1 - 9 steht; und
m für 0 - 4 steht; und die Verbindung von Formel (V) optional eine oder mehr Doppelbindung(en) enthalten kann und optional mit einer oder mehr (C1-C4)-Alkyl- oder bis zu C4-Alkenylgruppe(n) substituiert ist.

**8.** Pharmazeutische Zusammensetzung umfassend eine Verbindung mit der Strukturformel (I):

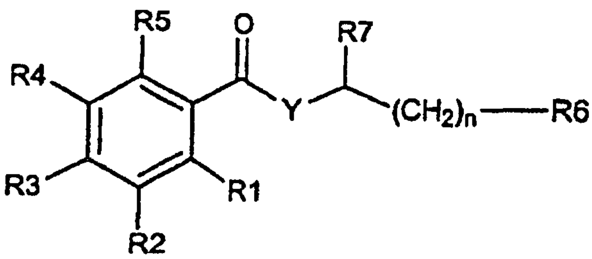

(I)

ein Stereoisomer davon, oder ein pharmazeutisch verträgliches Salz oder Hydrat davon oder einen physiologisch verträglichen und einen hydrolysierbaren Ester davon und ein(en) pharmazeutisch verträglichen/s Träger, Verdünnungsmittel oder Hilfsmittel; worin:

R1 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R2 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;

R3 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R4 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R5 für H, OH, Halogen, $(C_1-C_4)$-Alkyl, bis zu $C_4$-Alkenyl, $(C_1-C_4)$-Alkoxyoder $(C_1-C_4)$-Alkyl-COO- steht;
Y für O oder -NR' steht, worin R' für H oder $(C_1-C_4)$-Alkyl steht;
n für 0 - 8 steht;
R6 für einen 5- bis 8- gliedrigen Lacton- oder Lactamring steht;
R7 für H, $(C_1-C_4)$-Alkyl, bis zu $C_4$-Alkenyl, $(C_1-C_4)$-Alkoxy, Phenyl oder $(C_1-C_4)$-Alkyl-COO- steht;

worin der Lactam- oder Lactonring optional eine oder mehr C=C-Doppelbindung(en) umfasst und der Lactam- oder Lactonring optional mit einer oder mehr $(C_1-C_4)$-Alkyl- oder bis zu $C_4$-Alkenylgruppe(n) substituiert ist; für die Prophylaxe oder Behandlung eines Karzinoms und/oder einer malignen Erkrankung, oder einer Entzündungserkrankung oder medizinischen Erkrankung; oder für die Prophylaxe oder Behandlung einer Erkrankung oder medizinischen Erkrankung, ausgewählt aus der Gruppe, umfassend: eine akute und/oder chronische Entzündung, Autoimmunkrankheiten, Atemwegserkrankungen, Infektionskrankheiten, Transplantatabstoßung, neuronale Entzündung, Alzheimersche Krankheit und kardiovaskuläre Erkrankungen; oder für die Prophylaxe oder Behandlung von Onkogen-vermittelten Karzinomen oder malignen Erkrankungen; oder für die Prophylaxe oder Behandlung von Tumorwachstum und/oder einer Metastaseninvasion im Allgemeinen; oder für die Prävention oder Umkehr einer Multiarzneimittelresistenz.

**9.** Pharmazeutische Zusammensetzung, umfassend eine oder mehr Verbindung(en) nach einem der Ansprüche 1-6 und ein(en) pharmazeutisch verträglichen/s Träger, Verdünnungsmittel oder Hilfsmittel.

**10.** Zusammensetzung nach Anspruch 8 oder 9, die zusätzlich ein zytostatisches oder zytotoxisches Mittel umfasst.

**11.** Zusammensetzung nach Anspruch 8 oder 9, die zusätzlich ein Chemotherapeutikum umfasst.

**12.** Verwendung einer Verbindung mit der Strukturformel (I):

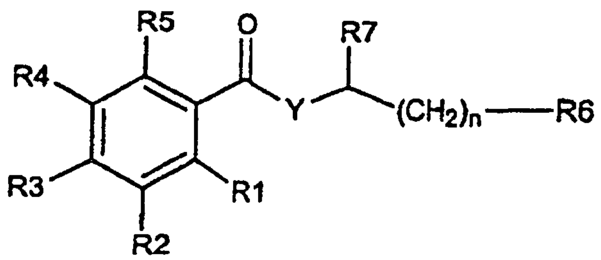

(I)

oder Stereoisomere davon, oder pharmazeutisch verträgliche Salze oder Hydrate davon oder physiologisch verträgliche und hydrolysierbare Ester davon, bei der Herstellung eines Arzneimittels für die Behandlung eines Säugers, worin:

R1 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R2 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R3 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R4 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R5 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
Y für O oder -NR' steht, worin R' für H oder $(C_1-C_4)$-Alkyl steht;
n für 0 - 8 steht;
R6 für einen 5- bis 8- gliedrigen Lacton- oder Lactamring steht;
R7 für H, $(C_1-C_4)$-Alkyl, bis zu $C_4$-Alkenyl, $(C_1-C_4)$-Alkoxy, Phenyl oder $(C_1-C_4)$ -Alkyl-COO- steht;

worin der Lactam- oder Lactonring optional eine oder mehr C=C-Doppelbindung(en) umfasst und der Lactam- oder Lactonring optional mit einer oder mehr $(C_1-C_4)$-Alkyl- oder bis zu $C_4$-Alkenylgruppe(n) substituiert ist.

**13.** Verwendung der Verbindung nach einem der Ansprüche 1 - 6 bei der Herstellung eines Arzneimittels für die Behandlung eines Säugers.

**14.** Verwendung nach Anspruch 12 oder 13, worin das genannte Arzneimittel in der Behandlung eines Karzinoms und/oder einer malignen Erkrankung verwendet wird.

**15.** Verwendung nach Anspruch 14, worin das genannte Karzinom und/oder die maligne Erkrankung eine abnorme Zellproliferation von malignen Zellen aus verschiedenen Geweben und/oder Organen umfasst, die aus der Gruppe ausgewählt sind, umfassend: Muskel-, Knochen- oder Bindegewebe, die Haut, das Hirn, die Lungen, die Geschlechtsorgane, das Lymph- oder Nierensystem, Mamma-oder Blutzellen, die Leber, den Verdauungstrakt, den Pankreas und die Schilddrüse und die Nebennieren.

**16.** Verwendung nach Anspruch 14, worin das genannte Karzinom und/oder die maligne Erkrankung aus der Gruppe ausgewählt ist, umfassend: solide Tumoren, Ovarial-, Mamma-, Hirn-, Prostata-, Kolon-, Magen-, Nieren- oder Hodenkarzinome, Kaposi-Sarkome, Cholangiome, Choriome, Neuroblastome, Wilms-Tumoren, Hodgkin-Lymphome, Melanome, multiple Myelome, lymphatische Leukämien und akute und/oder chronische granulozytäre Lymphome.

**17.** Verwendung nach Anspruch 12 oder 13, worin das genannte Arzneimittel in der Behandlung einer Entzündungserkrankung oder medizinischen Erkrankung verwendet wird.

**18.** Verwendung nach Anspruch 17, worin die genannte Erkrankung oder medizinische Erkrankung aus der Gruppe ausgewählt ist, umfassend: rheumatoide Arthritis, Osteoarthritis, septischer Schock, Psoriasis, Atherosklerose, entzündliche Darmerkrankung, Morbus Crohn und Asthma.

**19.** Verwendung nach Anspruch 12 oder 13, worin das Arzneimittel in der Behandlung einer Erkrankung oder medizinischen Erkrankung verwendet wird, die aus der Gruppe ausgewählt ist, umfassend: eine akute und/oder chronische Entzündung, Autoimmunerkrankungen, Atemwegserkrankungen, Infektionskrankheiten, Transplantatabstoßung, neuronale Entzündung, Alzheimersche Krankheit und kardiovaskuläre Erkrankungen.

**20.** Verwendung nach Anspruch 12 oder 13, worin das Arzneimittel in der Behandlung und Prävention von Onkogen-vermittelten Karzinomen und malignen Erkrankungen, für die Behandlung oder Prävention von Tumorwachstum und/oder Metastaseninvasion im Allgemeinen oder für die Prävention oder Umkehr einer Multiarzneimittelresistenz verwendet wird.

**21.** Verwendung nach einem der Ansprüche 12 - 20, worin der genannte Säuger ein Mensch ist.

**22.** Verbindung mit der Strukturformel (I):

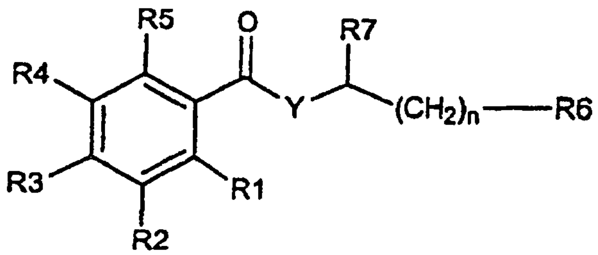

(I)

oder Stereoisomere davon, oder pharmazeutisch verträgliche Salze oder Hydrate davon oder physiologisch verträgliche und hydrolysierbare Ester davon, worin:

R1 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R2 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R3 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R4 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R5 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
Y für O oder -NR' steht, worin R' für H oder $(C_1-C_4)$-Alkyl steht;
n für 0 - 8 steht;

R6 für einen 5- bis 8- gliedrigen Lacton- oder Lactamring steht;
R7 für H, ($C_1$-$C_4$) -Alkyl, bis zu $C_4$-Alkenyl, ($C_1$-$C_4$)-Alkoxy, Phenyl oder ($C_1$-$C_4$)-Alkyl-COO- steht;

worin der Lactam- oder Lactonring optional eine oder mehr C=C-Doppelbindung(en) umfasst und der Lactam- oder Lactonring optional mit einer oder mehr ($C_1$-$C_4$)-Alkyl- oder bis zu $C_4$-Alkenylgruppe(n) substituiert ist;
für die Prophylaxe oder Behandlung eines Karzinoms und/oder einer malignen Erkrankung.

**23.** Verbindung nach einem der Ansprüche 1 - 6 für die Prophylaxe oder Behandlung eines Karzinoms und/oder einer malignen Erkrankung.

**24.** Verbindung nach Anspruch 22 oder 23, worin das genannte Karzinom und/oder die maligne Erkrankung eine abnorme Zellproliferation von malignen Zellen aus verschiedenen Geweben und/oder Organen umfasst, die aus der Gruppe ausgewählt sind, umfassend: Muskel-, Knochen- oder Bindegewebe, die Haut, das Hirn, die Lungen, die Geschlechtsorgane, das Lymph- oder Nierensystem, Mamma-oder Blutzellen, die Leber, den Verdauungstrakt, den Pankreas und die Schilddrüse oder die Nebennieren.

**25.** Verbindung nach Anspruch 22 oder 23, worin das genannte Karzinom und/oder die maligne Erkrankung aus der Gruppe ausgewählt sind, umfassend: solide Tumoren, Ovarial-, Mamma-, Hirn-, Prostata-, Kolon-, Magen-, Nieren- oder Hodenkarzinome, Kaposi-Sarkome, Cholangiome, Choriome, Neuroblastome, Wilms-Tumoren, Hodgkin-Lymphome, Melanome, multiple Myelome, lymphatische Leukämien und akute und/oder chronische granulozytäre Lymphome.

**26.** Verbindung mit der Strukturformel (I):

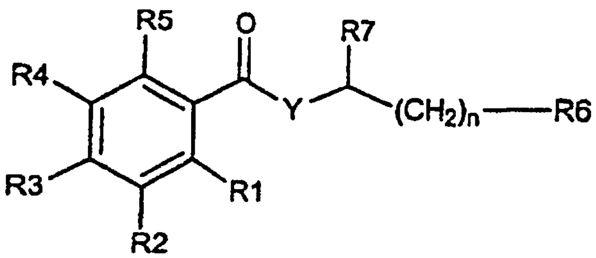

(I)

Stereoisomere davon, oder pharmazeutisch verträgliche Salze oder Hydrate davon oder physiologisch verträgliche und hydrolysierbare Ester davon, worin:

R1 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R2 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R3 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R4 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R5 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
Y für O oder -NR' steht, worin R' für H oder ($C_1$-$C_4$)-Alkyl steht;
n für 0 - 8 steht;
R6 für einen 5- bis 8- gliedrigen Lacton- oder Lactamring steht;
R7 für H, ($C_1$-$C_4$)-Alkyl, bis zu $C_4$-Alkenyl, ($C_1$-$C_4$)-Alkoxy, Phenyl oder ($C_1$-$C_4$)-Alkyl-COO- steht;

worin der Lactam- oder Lactonring optional eine oder mehr C=C-Doppelbindung(en) umfasst und der Lactam- oder Lactonring optional mit einer oder mehr ($C_1$-$C_4$)-Alkyl- oder bis zu $C_4$-Alkenylgruppe(n) substituiert ist;
für die Prophylaxe oder Behandlung einer Entzündungserkrankung oder medizinischen Erkrankung.

**27.** Verbindung nach einem der Ansprüche 1 - 6 für die Prophylaxe oder Behandlung einer Entzündungserkrankung oder medizinischen Erkrankung.

**28.** Verbindung nach Anspruch 26 oder 27, worin die genannte Erkrankung oder medizinische Erkrankung aus der Gruppe ausgewählt ist, umfassend: rheumatoide Arthritis, Osteoarthritis, septischer Schock, Psoriasis, Atherosklerose, entzündliche Darmerkrankung, Morbus Crohn und Asthma.

**29.** Verbindung mit der Strukturformel (I):

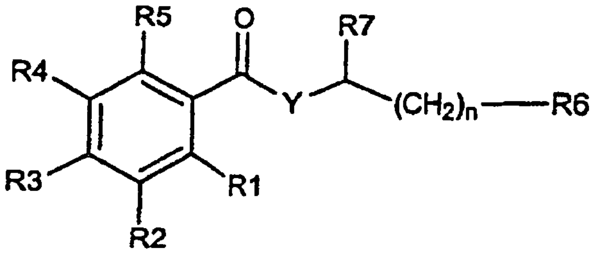

(I)

Stereoisomere davon, oder pharmazeutisch verträgliche Salze oder Hydrate davon oder physiologisch verträgliche und hydrolysierbare Ester davon, worin:

R1 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R2 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R3 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R4 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R5 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
Y für O oder -NR' steht, worin R' für H oder ($C_1$-$C_4$)-Alkyl steht;
n für 0 - 8 steht;
R6 für einen 5- bis 8- gliedrigen Lacton- oder Lactamring steht;
R7 für H, ($C_1$-$C_4$)-Alkyl, bis zu $C_4$-Alkenyl, ($C_1$-$C_4$)-Alkoxy, Phenyl oder ($C_1$-$C_4$)-Alkyl-COO- steht;

worin der Lactam- oder Lactonring optional eine oder mehr C=C-Doppelbindung(en) umfasst und der Lactam- oder Lactonring optional mit einer oder mehr ($C_1$-$C_4$)-Alkyl- oder bis zu $C_4$-Alkenylgruppe(n) substituiert ist, für die Behandlung und Prävention von Onkogen-vermittelten Karzinomen und malignen Erkrankungen, für die Behandlung oder Prävention von Tumorwachstum und/oder Metastaseninvasion im Allgemeinen oder für die Prävention oder Umkehr einer Multiarzneimittelresistenz.

**30.** Verbindung nach einem der Ansprüche 1 - 6 für die Behandlung und Prävention von Onkogen-vermittelten Karzinomen und malignen Erkrankungen, für die Behandlung oder Prävention von Tumorwachstum und/oder Metastaseninvasion im Allgemeinen oder für die Prävention oder Umkehr einer Multiarzneimittelresistenz.

**31.** Verbindung mit der Strukturformel (I):

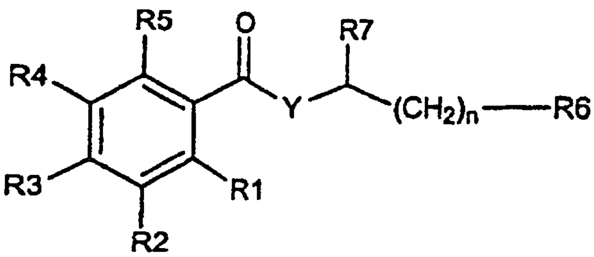

(I)

Stereoisomere davon, oder pharmazeutisch verträgliche Salze oder Hydrate davon oder physiologisch verträgliche und hydrolysierbare Ester davon, worin:

R1 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;
R2 für H, OH, Halogen, ($C_1$-$C_4$)-Alkyl, bis zu $C_4$-Alkenyl, ($C_1$-$C_4$)-Alkoxy oder ($C_1$-$C_4$)-Alkyl-COO- steht;

R3 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R4 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
R5 für H, OH, Halogen, $(C_1-C_4)$-Alkyl, bis zu $C_4$-Alkenyl, $(C_1-C_4)$-Alkoxy oder $(C_1-C_4)$-Alkyl-COO- steht;
Y für O oder -NR' steht, worin R' für H oder $(C_1-C_4)$-Alkyl steht;
n für 0 - 8 steht;
R6 für einen 5- bis 8- gliedrigen Lacton- oder Lactamring steht;
R7 für H, $(C_1-C_4)$-Alkyl, bis zu $C_4$-Alkenyl, $(C_1-C_4)$-Alkoxy, Phenyl oder $(C_1-C_4)$-Alkyl-COO- steht;

worin der Lactam- oder Lactonring optional eine oder mehr C=C-Doppelbindung(en) umfasst und der Lactam- oder Lactonring optional mit einer oder mehr $(C_1-C_4)$-Alkyl- oder bis zu $C_4$-Alkenylgruppe(n) substituiert ist;
für die Behandlung oder Prävention einer Erkrankung oder medizinischen Erkrankung, die aus der Gruppe ausgewählt ist, umfassend: eine akute und/oder chronische Entzündung, Autoimmunerkrankungen, Atemwegserkrankungen, Infektionskrankheiten, Transplantatabstoßung, neuronale Entzündung, Alzheimersche Krankheit und kardiovaskuläre Erkrankungen.

**32.** Verbindung nach einem der Ansprüche 1 - 6, für die Behandlung oder Prävention einer Erkrankung oder einer medizinischen Erkrankung, die aus der Gruppe ausgewählt ist, umfassend: eine akute und/oder chronische Entzündung, Autoimmunerkrankungen, Atemwegserkrankungen,
Infektionskrankheiten, Transplantatabstoßung, neuronale Entzündung, Alzheimersche Krankheit und kardiovaskuläre Erkrankungen.

**33.** Verbindung nach einem der Ansprüche 22 - 32 zur Behandlung eines Menschen.

## Revendications

**1.** Composé présentant la formule structurelle (I) :

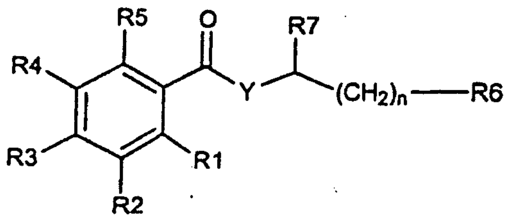

(I)

ou des stéréoisomères de celui-ci, ou des sels ou des hydrates pharmaceutiquement acceptables de celui-ci, ou des esters physiologiquement acceptables et hydrolysables de celui-ci, dans lequel :

R1 est H, OH, halogène, alkyle en $C_1$-$C_4$, alcényle jusqu'en $C_4$, alkoxy en $C_1$-$C_4$, ou alkyl-COO- en $C_1$-$C_4$ ;
R2 est H, OH, halogène, alkyle en $C_1$-$C_4$, alcényle jusqu'en $C_4$, alkoxy en $C_1$-$C_4$, ou alkyl-COO- en $C_1$-$C_4$ ;
R3 est H, OH, halogène, alkyle en $C_1$-$C_4$, alcényle jusqu'en $C_4$, alkoxy en $C_1$-$C_4$, ou alkyl-COO- en $C_1$-$C_4$ ;
R4 est H, OH, halogène, alkyle en $C_1$-$C_4$, alcényle jusqu'en $C_4$, alkoxy en $C_1$-$C_4$, ou alkyl-COO- en $C_1$-$C_4$ ;
R5 est H, OH, halogène, alkyle en $C_1$-$C_4$, alcényle jusqu'en $C_4$, alkoxy en $C_1$-$C_4$, ou alkyl-COO- en $C_1$-$C_4$ ;
Y est O, ou -NR' ; dans lequel R' est H ou alkyle en $C_1$-$C_4$ ;
n est 0-8 ;
R6 est un anneau lactone ou lactame à 5 à 8 chaînons ;
R7 est H, alkyle en $C_1$-$C_4$, alcényle jusqu'en $C_4$, alkoxy en $C_1$-$C_4$, phényle ou alkyl-COO- en $C_1$-$C_4$ ;

dans lequel l'anneau de lactame ou de lactone comprend éventuellement une ou plusieurs double liaisons C=C et l'anneau de lactame ou de lactone est éventuellement substitué par un ou plusieurs groupements alkyle en $C_1$-$C_4$, ou alcényle jusqu'en $C_4$ ;
à la condition que quand R1 = R2 = R3 = R4 = R5 = R7 = H, et Y est O, et n est 0, alors R6 est différent de

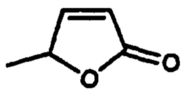 , 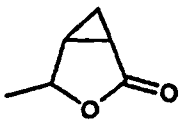 , 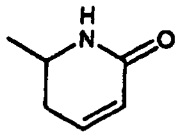 ou 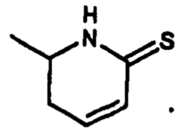 .

2. Composé selon la revendication 1, dans lequel :

R1 = H, OH, MeCOO- ou méthoxy ;
R2 = H ;
R3 = H, OH, MeCOO- ou méthoxy ;
R4 = H, méthoxy ou halogène ;
R5 = H, OH, méthoxy ou alkyle en $C_1$-$C_4$ ;
Y est O ;
R6 est

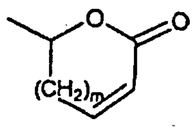

dans laquelle m est 0-4 ;
R7 = H, méthyle, isopropyle.

3. Composé selon la revendication 1, dans lequel :

R1 est H, -OH, MeO- ou MeCOO- ;
R2 est H ;
R3 est H, -OH, MeO- ou MeCOO- ;
R4 est H ou halogène ;
R5 est -OH, MeO-, MeCOO- ou méthyle.

4. Composé selon la revendication 2, dans lequel :

R1 est H ou MeO, R3 est H, -OH ou MeO- ; R4 est H et R5 est méthoxy ou méthyle.

5. Composé selon la revendication 1, dans lequel ledit composé est sélectionné parmi le groupe de composés :

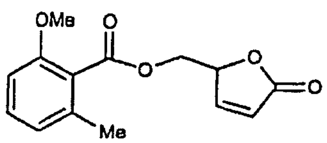 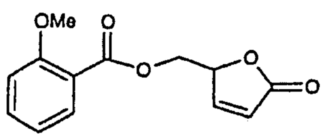 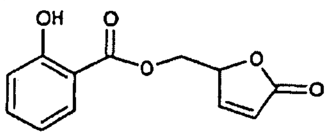

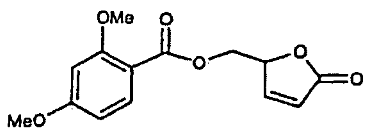 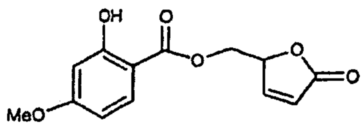 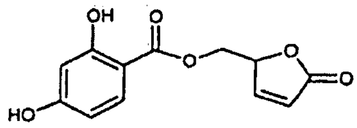

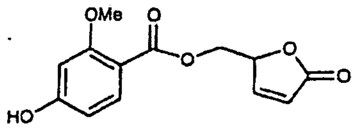 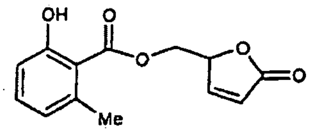

**6.** Composé selon la revendication 1, dans lequel ledit composé est sélectionné parmi le groupe de composés :

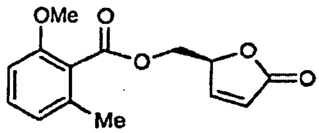 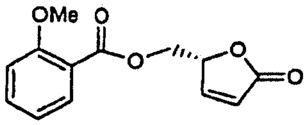 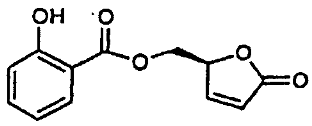

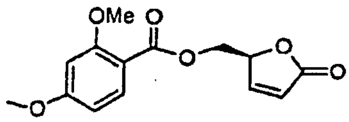 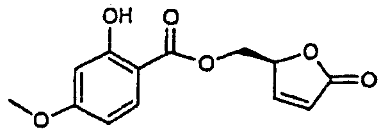 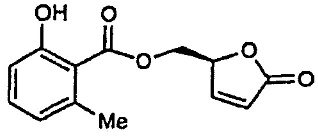

**7.** Processus de préparation d'un composé de formule I, selon la revendication 1, d'un ester ou d'un amide métaboliquement labile pharmaceutiquement acceptable de celui-ci, ou d'un sel pharmaceutiquement acceptable de celui-ci, qui comprend les étapes consistant à :

a) faire réagir un composé de formule (II):

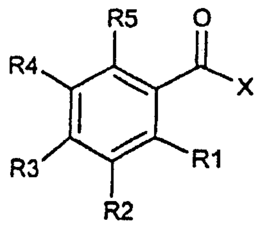

(II)

dans lequel :

R1 est H, OH, halogène, alkyle en $C_1$-$C_4$, alcényle jusqu'en $C_4$, alkoxy en $C_1$-$C_4$, ou alkyl-COO- en $C_1$-$C_4$ ;

R2 est H, OH, halogène, alkyle en $C_1$-$C_4$, alcényle jusqu'en $C_4$, alkoxy en $C_1$-$C_4$, ou alkyl-COO- en $C_1$-$C_4$ ;
R3 est H, OH, halogène, alkyle en $C_1$-$C_4$, alcényle jusqu'en $C_4$, alkoxy en $C_1$-$C_4$, ou alkyl-COO- en $C_1$-$C_4$ ;
R4 est H, OH, halogène, alkyle en $C_1$-$C_4$, alcényle jusqu'en $C_4$, alkoxy en $C_1$-$C_4$, ou alkyl-COO- en $C_1$-$C_4$ ; et
X est halogène ;
avec un composé de formule (III) :

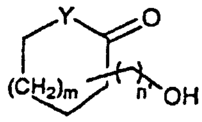

(III)

dans lequel :

n' est 1-9 ; et
m est 0-4 ;

le composé (III) contient éventuellement une ou plusieurs double liaisons C=C et est substitué par un ou plusieurs groupements alkyle en $C_1$-$C_4$, ou alcényle en $C_1$-$C_4$ ;
ou
b) faire réagir un composé de formule (IV) :

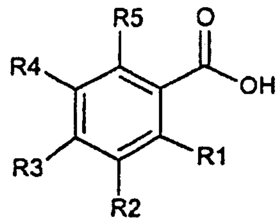

(IV)

dans lequel R1-R5 sont tels que définis ci-dessus ; avec un composé de formule (II) en présence d'un acide dans des conditions de distillation azéotropique ; ou
c) faire réagir un sel de métal alcalin ou alcalino-terreux du composé de formule (IV) :

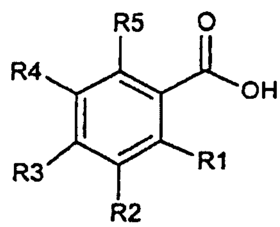

(IV)

dans lequel R1-R5 sont tels que définis ci-dessus ; avec un composé de formule (V) :

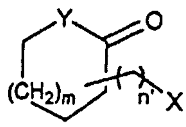

(V)

dans lequel :

X est F, Cl, Br ou I ;
n' est 1-9 ; et
m est 0-4 ; et le composé de formule (V) peut éventuellement contenir une ou plusieurs double liaisons et est éventuellement substitué par un ou plusieurs groupements alkyle en $C_1$-$C_4$, ou alcényle jusqu'en $C_4$.

**8.** Composition pharmaceutique comprenant un composé présentant la formule structurelle (I) :

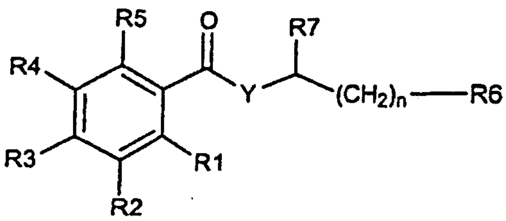

(I)

ou un stéréoisomère de celui-ci, ou un sel ou un hydrate pharmaceutiquement acceptable de celui-ci, ou un ester physiologiquement acceptable et hydrolysable de celui-ci, et un véhicule, un diluant ou un excipient pharmaceutiquement acceptable ;
dans lequel :

R1 est H, OH, halogène, alkyle en $C_1$-$C_4$, alcényle jusqu'en $C_4$, alkoxy en $C_1$-$C_4$, ou alkyl-COO- en $C_1$-$C_4$ ;
R2 est H, OH, halogène, alkyle en $C_1$-$C_4$, alcényle jusqu'en $C_4$, alkoxy en $C_1$-$C_4$, ou alkyl-COO- en $C_1$-$C_4$ ;
R3 est H, OH, halogène, alkyle en $C_1$-$C_4$, alcényle jusqu'en $C_4$, alkoxy en $C_1$-$C_4$, ou alkyl-COO- en $C_1$-$C_4$ ;
R4 est H, OH, halogène, alkyle en $C_1$-$C_4$, alcényle jusqu'en $C_4$, alkoxy en $C_1$-$C_4$, ou alkyl-COO- en $C_1$-$C_4$ ;
R5 est H, OH, halogène, alkyle en $C_1$-$C_4$, alcényle jusqu'en $C_4$, alkoxy en $C_1$-$C_4$, ou alkyl-COO- en $C_1$-$C_4$ ;
Y est O, ou -NR' ; dans lequel R' est H ou alkyle en $C_1$-$C_4$ ;
n est 0-8 ;
R6 est un anneau lactone ou lactame à 5 à 8 chaînons ;
R7 est H, alkyle en $C_1$-$C_4$, alcényle jusqu'en $C_4$, alkoxy en $C_1$-$C_4$, phényle ou alkyl-COO- en $C_1$-$C_4$ ;

dans lequel l'anneau de lactame ou de lactone comprend éventuellement une ou plusieurs double liaisons C=C et l'anneau de lactame ou de lactone est éventuellement substitué par un ou plusieurs groupements alkyle en $C_1$-$C_4$, ou alcényle jusqu'en $C_4$ ; pour la prophylaxie ou le traitement d'un cancer et/ou d'une maladie maligne, ou d'une maladie inflammatoire ou une condition médicale ; ou pour la prophylaxie ou le traitement d'une maladie ou d'une condition médicale sélectionnée parmi le groupe constitué de : inflammation aiguë et/ou chronique, maladies auto-immunes, maladies respiratoires, maladies infectieuses, rejet de greffe, inflammation neuronale, maladie d'Alzheimer et maladies cardiovasculaires ; ou pour la prophylaxie ou le traitement de cancers médiés par un oncogène ou de maladies malignes ; ou pour la prophylaxie ou le traitement de croissance de tumeurs et/ou de l'invasion de métastases en général ; ou pour la prévention ou l'inversion d'une résistance multiple aux médicaments.

**9.** Composition pharmaceutique comprenant un ou plusieurs composés selon l'une quelconque des revendications 1 à 6 et un véhicule, un diluant ou un excipient pharmaceutiquement acceptable.

**10.** Composition selon la revendication 8 ou la revendication 9, comprenant en outre un agent cytostatique ou cytotoxique.

**11.** Composition selon la revendication 8 ou la revendication 9, comprenant en outre un agent chimiothérapeutique.

**12.** Utilisation d'un composé présentant la formule structurelle (I) :

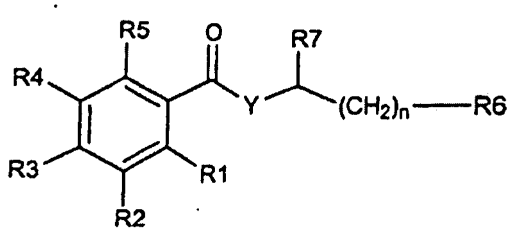

(I)

ou des stéréoisomères de celui-ci, ou des sels ou des hydrates pharmaceutiquement acceptables de celui-ci, ou des esters physiologiquement acceptables et hydrolysables de celui-ci, dans la fabrication d'un médicament pour le traitement d'un mammifère, dans lequel :

R1 est H, OH, halogène, alkyle en $C_1$-$C_4$, alcényle jusqu'en $C_4$, alkoxy en $C_1$-$C_4$, ou alkyl-COO- en $C_1$-$C_4$ ;
R2 est H, OH, halogène, alkyle en $C_1$-$C_4$, alcényle jusqu'en $C_4$, alkoxy en $C_1$-$C_4$, ou alkyl-COO- en $C_1$-$C_4$ ;
R3 est H, OH, halogène, alkyle en $C_1$-$C_4$, alcényle jusqu'en $C_4$, alkoxy en $C_1$-$C_4$, ou alkyl-COO- en $C_1$-$C_4$ ;
R4 est H, OH, halogène, alkyle en $C_1$-$C_4$, alcényle jusqu'en $C_4$, alkoxy en $C_1$-$C_4$, ou alkyl-COO- en $C_1$-$C_4$ ;
R5 est H, OH, halogène, alkyle en $C_1$-$C_4$, alcényle jusqu'en $C_4$, alkoxy en $C_1$-$C_4$, ou alkyl-COO- en $C_1$-$C_4$ ;
Y est O, ou -NR' ; dans lequel R' est H ou alkyle en $C_1$-$C_4$ ;
n est 0-8 ;
R6 est un anneau lactone ou lactame à 5 à 8 chaînons ;
R7 est H, alkyle en $C_1$-$C_4$, alcényle jusqu'en $C_4$, alkoxy en $C_1$-$C_4$, phényle ou alkyl-COO- en $C_1$-$C_4$ ;
dans lequel l'anneau de lactame ou de lactone comprend éventuellement une ou plusieurs double liaisons C=C et l'anneau de lactame ou de lactone est éventuellement substitué par un ou plusieurs groupements alkyle en $C_1$-$C_4$, ou alcényle jusqu'en $C_4$.

**13.** Utilisation du composé selon l'une quelconque des revendications 1 à 6 dans la fabrication d'un médicament pour le traitement d'un mammifère.

**14.** Utilisation selon la revendication 12 ou la revendication 13, dans laquelle ledit médicament est utilisé dans le traitement d'un cancer et/ou d'une maladie maligne.

**15.** Utilisation selon la revendication 14, dans laquelle ledit cancer et/ou ladite maladie maligne comprend une prolifération cellulaire anormale de cellules malignes de divers tissus et/ou organes sélectionnés parmi le groupe constitué de : muscle, os ou tissus conjonctifs, la peau, le cerveau, les poumons, les organes sexuels, le système lymphatique ou le système rénal, les cellules mammaires ou sanguines, le foie, le tube digestif, le pancréas et la glande thyroïde ou la glande surrénale.

**16.** Utilisation selon la revendication 14, dans laquelle ledit cancer et/ou ladite maladie maligne est sélectionné parmi le groupe constitué de : tumeurs solides, cancers de l'ovaire, du sein, du cerveau, de la prostate, du colon, de l'estomac, du rein ou des testicules, sarcome de Kaposi, cholangiome, choriome, neuroblastome, tumeur de Wilms, maladie d'Hodgkin, mélanomes, myélomes multiples, leucémies lymphatiques et lymphomes granulocytiques aigus et/ou chroniques.

**17.** Utilisation selon la revendication 12 ou la revendication 13, dans laquelle ledit médicament est utilisé dans le traitement d'une maladie inflammatoire ou d'une condition médicale.

**18.** Utilisation selon la revendication 17, dans laquelle ladite maladie ou condition médicale est sélectionnée parmi le groupe constitué de : arthrite rhumatoïde, arthrose, choc septique, psoriasis, athérosclérose, maladie intestinale inflammatoire, maladie de Crohn et asthme.

**19.** Utilisation selon la revendication 12 ou la revendication 13, dans laquelle le médicament est utilisé dans le traitement d'une maladie ou d'une condition médicale sélectionnée parmi le groupe constitué de : inflammation aiguë et/ou chronique, maladies auto-immunes, maladies respiratoires, maladies infectieuses, rejet de greffe, inflammation neuronale, maladie d'Alzheimer et maladies cardiovasculaires.

**20.** Utilisation selon la revendication 12 ou la revendication 13, dans laquelle le médicament est utilisé dans le traitement et la prévention de cancers médiés par un oncogène et de maladies malignes, pour le traitement ou la prévention de la croissance de tumeurs et/ou de l'invasion de métastases en général,
ou pour la prévention ou l'inversion d'une résistance multiple aux médicaments.

**21.** Utilisation selon l'une quelconque des revendications 12 - 20, dans laquelle ledit mammifère est un humain.

**22.** Composé présentant la formule structurelle (I) :

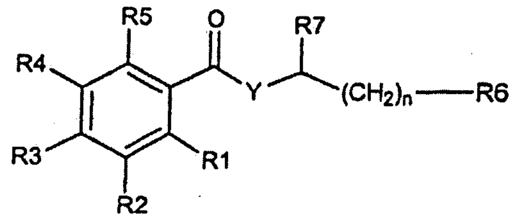

(I)

ou des stéréoisomères de celui-ci, ou des sels ou des hydrates pharmaceutiquement acceptables de celui-ci, ou des esters physiologiquement acceptables et hydrolysables de celui-ci, dans lequel :

R1 est H, OH, halogène, alkyle en $C_1$-$C_4$, alcényle jusqu'en $C_4$, alkoxy en $C_1$-$C_4$, ou alkyl-COO- en $C_1$-$C_4$ ;
R2 est H, OH, halogène, alkyle en $C_1$-$C_4$, alcényle jusqu'en $C_4$, alkoxy en $C_1$-$C_4$, ou alkyl-COO- en $C_1$-$C_4$ ;
R3 est H, OH, halogène, alkyle en $C_1$-$C_4$, alcényle jusqu'en $C_4$, alkoxy en $C_1$-$C_4$, ou alkyl-COO- en $C_1$-$C_4$ ;
R4 est H, OH, halogène, alkyle en $C_1$-$C_4$, alcényle jusqu'en $C_4$, alkoxy en $C_1$-$C_4$, ou alkyl-COO- en $C_1$-$C_4$ ;
R5 est H, OH, halogène, alkyle en $C_1$-$C_4$, alcényle jusqu'en $C_4$, alkoxy en $C_1$-$C_4$, ou alkyl-COO- en $C_1$-$C_4$ ;
Y est O, ou -NR' ; dans lequel R' est H ou alkyle en $C_1$-$C_4$ ;
n est 0-8 ;
R6 est un anneau lactone ou lactame à 5 à 8 chaînons ;
R7 est H, alkyle en $C_1$-$C_4$, alcényle jusqu'en $C_4$, alkoxy en $C_1$-$C_4$, phényle ou alkyl-COO- en $C_1$-$C_4$ ;

dans lequel l'anneau de lacame ou de lactone comprend éventuellement une ou plusieurs double liaisons C=C et l'anneau de lactame ou de lactone est éventuellement substitué par un ou plusieurs groupements alkyle en $C_{1-}C_4$, ou alcényle jusqu'en $C_4$ ;
pour la prophylaxie ou le traitement d'un cancer et/ou d'une maladie maligne.

**23.** Composé selon l'une quelconque des revendications 1 - 6, pour la prophylaxie ou le traitement d'un cancer et/ou d'une maladie maligne.

**24.** Composé selon la revendication 22 ou la revendication 23, dans lequel ledit cancer et/ou ladite maladie maligne comprend une prolifération cellulaire anormale de cellules malignes de divers tissus et/ou organes sélectionnés parmi le groupe constitué de : muscle, os
ou tissus conjonctifs, la peau, le cerveau, les poumons, les organes sexuels, le système lymphatique
ou le système rénal, les cellules mammaires ou sanguines, le foie, le tube digestif, le pancréas et la glande thyroïde ou la glande surrénale.

**25.** Composé selon la revendication 22 ou la revendication 23, dans lequel ledit cancer et/ou ladite maladie maligne est sélectionné parmi le groupe constitué de :

tumeurs solides, cancers de l'ovaire, du sein, du cerveau, de la prostate, du colon, de l'estomac, du rein ou des testicules, sarcome de Kaposi, cholangiome, choriome, neuroblastome, tumeur de Wilms, maladie d'Hodgkin, mélanomes, myélomes multiples, leucémies lymphatiques et lymphomes granulocytiques aigus et/ou chroniques.

**26.** Composé présentant la formule structurelle (I) :

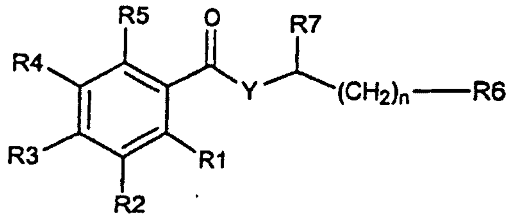

(I)

des stéréoisomères de celui-ci, ou des sels ou des hydrates pharmaceutiquement acceptables de celui-ci, ou des esters physiologiquement acceptables et hydrolysables de celui-ci, dans lequel :

R1 est H, OH, halogène, alkyle en $C_1$-$C_4$, alcényle jusqu'en $C_4$, alkoxy en $C_1$-$C_4$, ou alkyl-COO- en $C_1$-$C_4$ ;
R2 est H, OH, halogène, alkyle en $C_1$-$C_4$, alcényle jusqu'en $C_4$, alkoxy en $C_1$-$C_4$, ou alkyl-COO- en $C_1$-$C_4$ ;
R3 est H, OH, halogène, alkyle en $C_1$-$C_4$, alcényle jusqu'en $C_4$, alkoxy en $C_1$-$C_4$, ou alkyl-COO- en $C_1$-$C_4$ ;
R4 est H, OH, halogène, alkyle en $C_1$-$C_4$, alcényle jusqu'en $C_4$, alkoxy en $C_1$-$C_4$, ou alkyl-COO- en $C_1$-$C_4$ ;
R5 est H, OH, halogène, alkyle en $C_1$-$C_4$, alcényle jusqu'en $C_4$, alkoxy en $C_1$-$C_4$, ou alkyl-COO- en $C_1$-$C_4$ ;
Y est O, ou -NR' ; dans lequel R' est H ou alkyle en $C_1$-$C_4$ ;
n est 0-8 ;
R6 est un anneau lactone ou lactame à 5 à 8 chaînons ;
R7 est H, alkyle en $C_1$-$C_4$, alcényle jusqu'en $C_4$, alkoxy en $C_1$-$C_4$, phényle ou alkyl-COO- en $C_1$-$C_4$ ;

dans lequel l'anneau de lactame ou de lactone comprend éventuellement une ou plusieurs double liaisons C=C et l'anneau de lactame ou de lactone est éventuellement substitué par un ou plusieurs groupements alkyle en $C_1$-$C_4$, ou alcényle jusqu'en $C_4$ ;
pour la prophylaxie ou le traitement d'une maladie inflammatoire ou d'une condition médicale.

**27.** Composé selon l'une quelconque des revendications 1 - 6, pour la prophylaxie ou le traitement d'une maladie inflammatoire ou d'une condition médicale.

**28.** Composé selon la revendication 26 ou la revendication 27, dans lequel ladite maladie ou condition médicale est sélectionnée parmi le groupe constitué de : arthrite rhumatoïde, arthrose, choc septique, psoriasis, athérosclérose, maladie intestinale inflammatoire, maladie de Crohn et asthme.

**29.** Composé présentant la formule structurelle (I) :

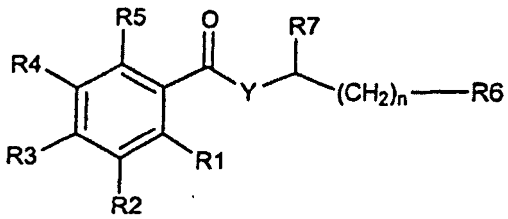

(I)

des stéréoisomères de celui-ci, ou des sels ou des hydrates pharmaceutiquement acceptables de celui-ci, ou des esters physiologiquement acceptables et hydrolysables de celui-ci, dans lequel :

R1 est H, OH, halogène, alkyle en $C_1$-$C_4$, alcényle jusqu'en $C_4$, alkoxy en $C_1$-$C_4$, ou alkyl-COO- en $C_1$-$C_4$ ;
R2 est H, OH, halogène, alkyle en $C_1$-$C_4$, alcényle jusqu'en $C_4$, alkoxy en $C_1$-$C_4$, ou alkyl-COO- en $C_1$-$C_4$ ;
R3 est H, OH, halogène, alkyle en $C_1$-$C_4$, alcényle jusqu'en $C_4$, alkoxy en $C_1$-$C_4$, ou alkyl-COO- en $C_1$-$C_4$ ;

R4 est H, OH, halogène, alkyle en $C_1$-$C_4$, alcényle jusqu'en $C_4$, alkoxy en $C_1$-$C_4$, ou alkyl-COO- en $C_1$-$C_4$ ;
R5 est H, OH, halogène, alkyle en $C_1$-$C_4$, alcényle jusqu'en $C_4$, alkoxy en $C_1$-$C_4$, ou alkyl-COO- en $C_1$-$C_4$ ;
Y est O, ou -NR' ; dans lequel R' est H ou alkyle en $C_1$-$C_4$ ;
n est 0-8 ;
R6 est un anneau lactone ou lactame à 5 à 8 chaînons ;
R7 est H, alkyle en $C_1$-$C_4$, alcényle jusqu'en $C_4$, alkoxy en $C_1$-$C_4$, phényle ou alkyl-COO- en $C_1$-$C_4$ ;

dans lequel l'anneau de lactame ou de lactone comprend éventuellement une ou plusieurs double liaisons C=C et l'anneau de lactame ou de lactone est éventuellement substitué par un ou plusieurs groupements alkyle en $C_1$-$C_4$, ou alcényle jusqu'en $C_4$, pour le traitement et la prévention de cancers médiés par un oncogène ou de maladies malignes, pour le traitement ou la prévention de croissance de tumeurs et/ou de l'invasion de métastases en général, ou pour la prévention ou l'inversion d'une résistance multiple aux médicaments.

**30.** Composé selon l'une quelconque des revendications 1 - 6, pour le traitement et la prévention de cancers médiés par un oncogène ou de maladies malignes, pour le traitement ou la prévention de croissance de tumeurs et/ou de l'invasion de métastases en général, ou pour la prévention ou l'inversion d'une résistance multiple aux médicaments.

**31.** Composé présentant la formule structurelle (I) :

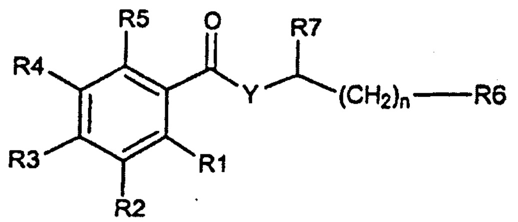

(I)

des stéréoisomères de celui-ci, ou des sels ou des hydrates pharmaceutiquement acceptables de celui-ci, ou des esters physiologiquement acceptables et hydrolysables de celui-ci, dans lequel :

R1 est H, OH, halogène, alkyle en $C_1$-$C_4$, alcényle jusqu'en $C_4$, alkoxy en $C_1$-$C_4$, ou alkyl-COO- en $C_1$-$C_4$ ;
R2 est H, OH, halogène, alkyle en $C_1$-$C_4$, alcényle jusqu'en $C_4$, alkoxy en $C_1$-$C_4$, ou alkyl-COO- en $C_1$-$C_4$ ;
R3 est H, OH, halogène, alkyle en $C_1$-$C_4$, alcényle jusqu'en $C_4$, alkoxy en $C_1$-$C_4$, ou alkyl-COO- en $C_1$-$C_4$ ;
R4 est H, OH, halogène, alkyle en $C_1$-$C_4$, alcényle jusqu'en $C_4$, alkoxy en $C_1$-$C_4$, ou alkyl-COO- en $C_1$-$C_4$ ;
R5 est H, OH, halogène, alkyle en $C_1$-$C_4$, alcényle jusqu'en $C_4$, alkoxy en $C_1$-$C_4$, ou alkyl-COO- en $C_1$-$C_4$ ;
Y est O, ou -NR' ; dans lequel R' est H ou alkyle en $C_1$-$C_4$ ;
n est 0-8 ;
R6 est un anneau lactone ou lactame à 5 à 8 chaînons ;
R7 est H, alkyle en $C_1$-$C_4$, alcényle jusqu'en $C_4$, alkoxy en $C_1$-$C_4$, phényle ou alkyl-COO- en $C_1$-$C_4$ ;

dans lequel l'anneau de lactame ou de lactone comprend éventuellement une ou plusieurs double liaisons C=C et l'anneau de lactame ou de lactone est éventuellement substitué par un ou plusieurs groupements alkyle en $C_1$-$C_4$, ou alcényle jusqu'en $C_4$, pour le traitement ou la prévention d'une maladie ou d'une condition médicale sélectionnée parmi le groupe constitué de : inflammation aiguë et/ou chronique, maladies auto-immunes, maladies respiratoires, maladies infectieuses, rejet de greffe, inflammation neuronale, maladie d'Alzheimer et maladies cardiovasculaires.

**32.** Composé selon l'une quelconque des revendications 1 - 6, pour le traitement ou la prévention d'une maladie ou d'une condition médicale sélectionnée parmi le groupe constitué de : inflammation aiguë et/ou chronique, maladies auto-immunes, maladies respiratoires, maladies infectieuses, rejet de greffe, inflammation neuronale, maladie d'Alzheimer et maladies cardiovasculaires.

**33.** Composé selon l'une quelconque des revendications 22 - 32, pour le traitement d'un humain.

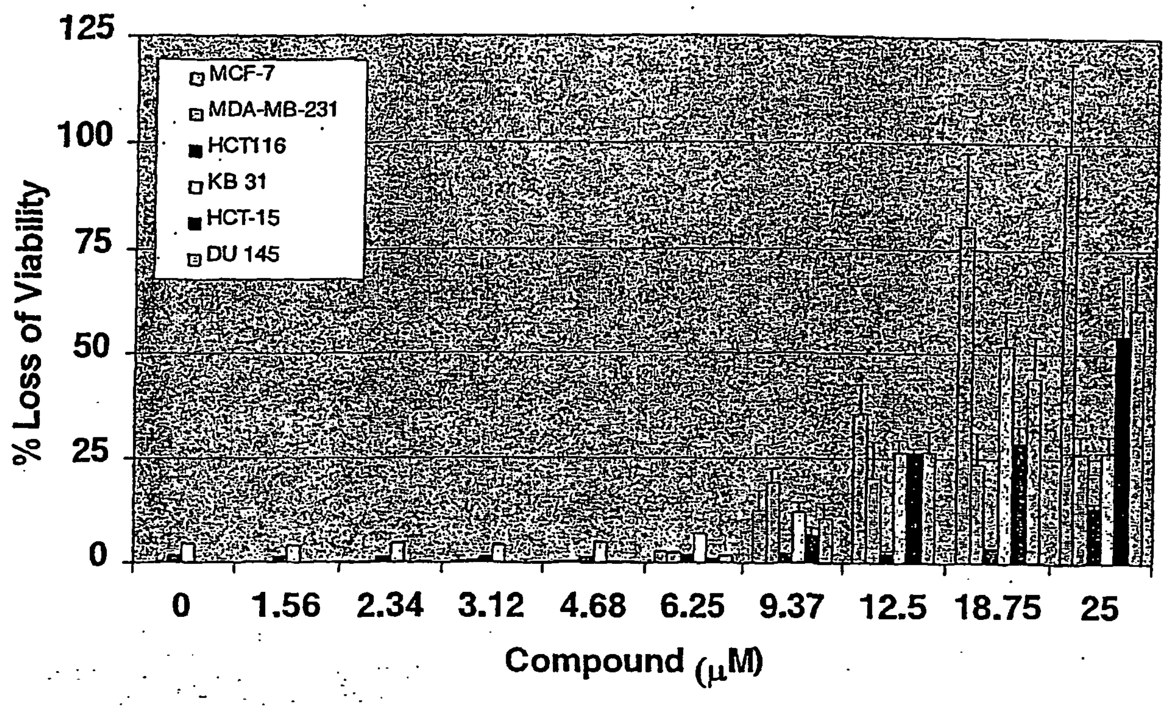
MCF-7
MDA-MB-231
HCT116
KB 31
HCT-15
DU 145
% Loss of Viability
125
100
75
50
25
0
0
1.56
2.34
3.12
4.68
6.25
9.37
12.5
18.75
25
Compound (μM)

Figure 1

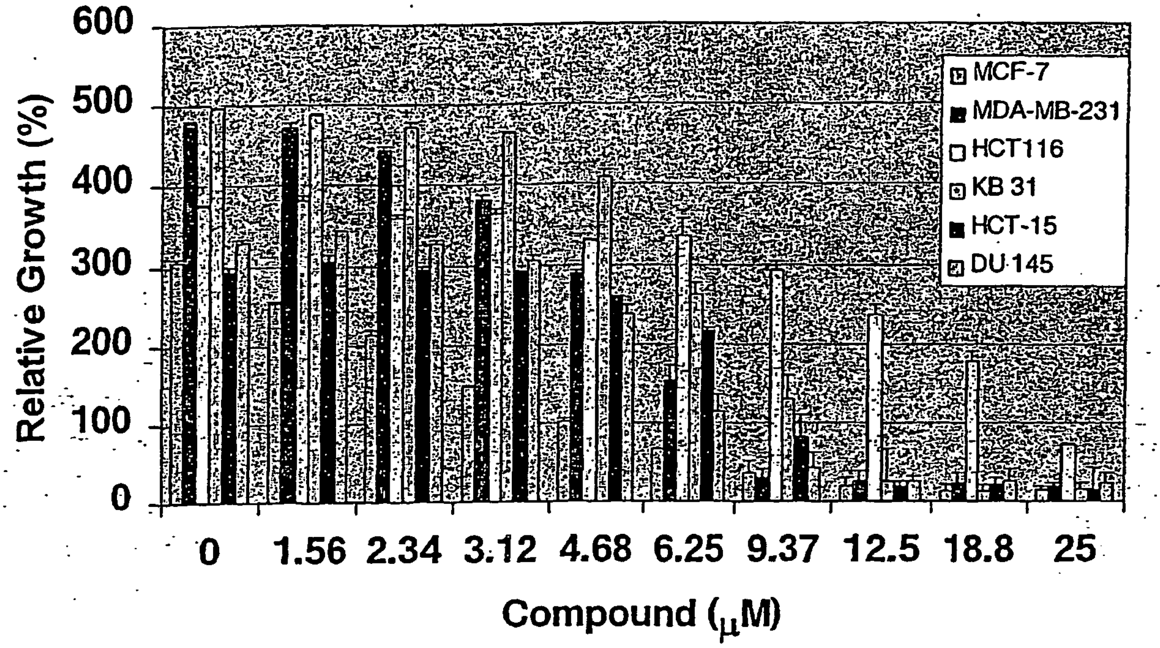
600
500
400
300
200
100
0
Relative Growth (%)
MCF-7
MDA-MB-231
HCT116
KB 31
HCT-15
DU 145
0
1.56
2.34
3.12
4.68
6.25
9.37
12.5
18.8
25
Compound (μM)

Figure 2